# EUROPÄISCHE PATENTSCHRIFT

(11) **EP 2 965 783 B1**
(45) Veröffentlichungstag und Bekanntmachung des Hinweises auf die Patenterteilung: **31.01.2018**
(21) Anmeldenummer: 15173082.7
(22) Anmeldetag: 22.06.2015
(51) Int. Cl.: B23K 1/00, B23K 31/02, C25D 7/06, A61N 1/375

(54) **VERFAHREN ZUR HERSTELLUNG EINES STIFTS FÜR EINE DURCHFÜHRUNG EINES ELEKTROMEDIZINISCHEN IMPLANTATS UND EINER DURCHFÜHRUNG**
METHOD FOR PRODUCING A PIN FOR A FEEDTHROUGH OF AN ELECTROMEDICAL IMPLANT AND A FEEDTHROUGH
PROCÉDÉ DE FABRICATION D'UNE TIGE POUR UNE CONDUITE D'UN IMPLANT ÉLECTROMEDICAL ET CONDUITE

(30) Priorität: 07.07.2014 US 201462021212 P
(43) Veröffentlichungstag der Anmeldung: 13.01.2016
(73) Patentinhaber: BIOTRONIK SE & Co. KG, 12359 Berlin (DE)
(72) Erfinder: Kronmüller, Daniel, 90409 Nürnberg (DE); Arnold, Michael, 91056 Erlangen (DE); Teske, Josef, 96103 Hallstadt (DE); Meidlein, Peter, 90443 Nürnberg (DE)
(74) Vertreter: Galander, Marcus

(56) Entgegenhaltungen:
- US-A- 3 545 606
- US-A- 4 729 504
- US-A1- 2005 060 003
- US-A1- 2008 257 581
- US-A1- 2012 200 011

## Beschreibung

Die vorliegende Erfindung betrifft ein Verfahren zur Herstellung eines Stifts für ein elektromedizinisches Implantat, ein Verfahren zur Herstellung einer Durchführung und eines elektromedizinisches Implantats sowie einen entsprechend hergestellten Stift, eine entsprechend hergestellte Durchführung und Implantat.

Medizinische oder aktive Implantate sind in großer Vielfalt aus dem Stand der Technik bekannt. Als elektromedizinisches Implantat wird im Zusammenhang mit der vorliegenden Erfindung ein Implantat verstanden, das eine Stromversorgung (z. B. Batterie) und elektrische und/oder elektronische Bauteile wie eine Platine umfasst, welche in einem Gehäuse angeordnet sind, das hermetisch dicht abgeschlossen ist. Derartige elektromedizinische Implantate sind beispielsweise Herzschrittmacher, Defibrillatoren, Neurostimulatoren, Leadless Pacemaker, Cardioverter, Medikamentenpumpenimplantate, Cochlea-Implantate oder andere hermetisch gekapselte elektronische Produkte zur Implantation in einen menschlichen oder tierischen Körper.

Häufig sind derartige Implantate mit Elektrodenleitungen verbunden, welche nach der Implantation in einen menschlichen oder tierischen Körper diesen therapieren, beispielsweise Stimulationsimpulse und/oder Defibrillatorschocks an bestimmte Körperstellen übertragen und/oder abgeben, oder dazu dienen, elektrische Potenziale von Körperstellen zu erfassen. Hierfür muss eine elektrische Verbindung zwischen den im Gehäuseinneren angeordneten elektrischen und/oder elektronischen Bauteilen und der jeweiligen Elektrodenleitung hergestellt werden. Diese elektrische Verbindung wird in der Regel mittels einer Durchführung und/oder einem sogenannten Header realisiert. Eine derartige Durchführung sorgt dabei für mindestens eine elektrische Verbindung zwischen dem Inneren des Gehäuses und dem Äußeren und schließt gleichzeitig das Gehäuse des Implantats hermetisch ab. Der über der Durchführung befestigte Header führt die elektrische Verbindung der Durchführung weiter zu einer Kontaktstelle und dient zum Einstecken der mindestens einen Elektrodenleitung in eine entsprechende, meist genormte Buchse. An den Kontaktstellen der Buchse wird so ein elektrischer Kontakt zwischen dem Implantat und dem Anschlussstück der Elektrodenleitung hergestellt. Eine Durchführung und ein Header können auch in einem einzigen Bauteil realisiert sein. Auch in diesem Fall wird ein solches kombiniertes Bauteil im Folgenden allgemein als Durchführung bezeichnet.

In der Regel weisen derartige Durchführungen einen elektrisch isolierenden Körper, den Isolator, auf, der häufig aus einer Keramik gefertigt ist und den hermetischen Abschluss des Gehäuses realisiert. Hierfür hat der Isolator oft einen Flansch, mit dem der Isolator in das offene Ende des Gehäuses des Implantats eingesetzt wird. In dem Isolator sind ferner häufig durchgehende Ausnehmungen, z.B. Bohrungen, enthalten, in denen jeweils ein Anschlussstift (im Folgenden kurz Stift) angeordnet ist, der auch als Terminal Pin oder kurz Pin bezeichnet wird. Der Stift ist häufig mittels Hochtemperaturlöten in der Ausnehmung befestigt, welche zusätzlich eine Durchführungshülse aufweisen kann. Der Stift dient der Herstellung einer elektrischen Verbindung zwischen dem Gehäuseinneren und dem Header bzw. der Elektrodenleitung. Eine derartige Durchführung mit Stift sowie ein Herstellverfahren einer solchen Durchführung sind aus der Druckschrift US 3,545,606 bekannt. Ferner ist beispielsweise aus der Druckschrift EP 2 371 418 A2 eine Durchführung mit einem Anschlussstift bekannt, deren Stift einen ersten Abschnitt aus einem biokompatiblen Material und einen zweiten Abschnitt aus einem Material, das mit niedriger Energie fügbar ist, aufweist. Der zweite Abschnitt soll dabei im Innern des Gehäuses des Implantats angeordnet werden.

Löten ist ein bekanntes thermisches Verfahren zum stoffschlüssigen Fügen von Werkstoffen, mit dem eine elektrische Verbindung hergestellt werden kann und das unter Verwendung eines Lots durchgeführt wird. In Abhängigkeit von der Temperatur unterscheidet der Fachmann drei bekannte Lötverfahren. Im Temperaturbereich bis 450°C spricht man vom Weichlöten. Bekannte Weichlote sind z.B. Sn63Pb37, Sn96Ag4, Au80Sn20. Beim Löten im Temperaturbereich zwischen 450°C und 900°C spricht man vom Hartlöten. Es werden häufig Silber- bzw. Messinglote eingesetzt (z.B. L-Ag44 (Ag44Cu30Zn26)). Bei Temperaturen oberhalb von 900°C spricht man von Hochtemperaturlöten. In der Medizintechnik verwendete Hochtemperaturlote sind zum Beispiel Au(99,95), AuAg8, AuPt10, Ti60Ni25Cu15.

Konventionell wird der in der Durchführung angeordnete Stift direkt mit einem Anschluss einer Platine mittels Weichlöten oder Schweißen verbunden, um die elektrische Verbindung mit dem auf der Platine angeordneten elektronischen Schaltkreis herzustellen. In der Druckschrift EP 2 529 790 A1 wird die Verwendung eines Verbinders offenbart, der mittels einer Klippverbindung an dem Anschlussstift befestigt wird. Der Verbinder weist zudem eine Hülse auf, die den Anschlussstift umgibt und zur Herstellung einer elektrischen Verbindung mittels Weichlöten oder Schweißen auf einer im Innern des Implantats angeordneten Platine festgelegt wird.

Bei der Herstellung derartiger Durchführungen und Implantate sind insbesondere das Einsetzen und Verlöten des Stifts sowie die Herstellung der elektrischen Verbindung zwischen Stift und Platine aufwändig und kostenintensiv. Die Elemente eines Stifts werden zunächst einzeln gefertigt und danach manuell zusammengesetzt und gefügt.

Beispielsweise werden Goldlotringe oder -hülsen vor dem Hartverlöten des Stifts mit der Durchführung separat gefertigt und von Hand einzeln mit einem Stift zusammengesetzt. Hierbei besteht das Problem, dass, wenn ein Lotring oder eine Lothülse während des Bestückens abfällt, die gesamte Durchführung entstückt werden muss. Bei mehrpoligen Durchführungen sind daher die Kosten durch Bestückungsfehler sehr hoch. Weiter besteht das Problem, dass die Warengruppen Stift und Lot einzeln im Wareneingang des produzierenden Unternehmens ankommen. Diese Warengruppen müssen bis zur Verarbeitung separat von anderen Komponenten gehalten werden. Daher entsteht in der Materialwirtschaft ebenfalls ein hoher Aufwand je Komponente. Zudem muss jede Komponente unabhängig auf Fehler geprüft werden. Der Aufwand für einzelne, damit im Zusammenhang stehende Prozesse (Stanzen, Reinigen, Sortieren, Verpacken) übersteigen den Materialwert des jeweiligen Bauteils um ein Vielfaches.

Das Lot kann durch einen vorgelagerten Hochtemperaturlötprozess auf den Drahtstift gelötet werden. Hierbei handelt es sich jedoch um einen mehrstufigen Fügeprozess aus den Einzelkomponenten, bei dem es insgesamt zu keiner Arbeitszeit- oder Kosteneinsparung kommt.

Weiter besteht die Möglichkeit, Stifte galvanisch oder mittels Beschichtungsverfahren mit einem Lotmaterial zu beschichten. Beispielsweise offenbart die Druckschrift US 2008/0257581 A1 ein Verfahren zur Herstellung eines Stifts für eine Durchführung, bei dem zuerst ein folien-, blech- oder bandförmiges Halbzeug durch Beschichten eines Kupfer enthaltenden Bleches mit einem zweiten, Nickel enthaltenden Schichtelement erstellt wird, und anschließend ein Stift oder ein Satz bestehend aus mehreren, mit einem Verbindungssteg verbundenen Stiften von dem Halbzeug zumindest teilweise abtrennt wird.

Das galvanische Beschichten von Drahtabschnitten als Schüttgut ist jedoch ebenfalls sehr aufwändig, da eine gleichmäßige Schichtdicke nur durch vorherige Vereinzelung und Ausrichtung der Stifte gewährleistet werden kann. Weiter kann es zu Einlagerungen aus der Galvaniklösung kommen. Eine derartige Galvaniklösung stellt zudem häufig einen Gefahr- oder Giftstoff dar, welcher im Bereich der Medizintechnik unerwünscht ist und deren Entsorgung problematisch sein kann.

Werden PVD- beziehungsweise CVD-Verfahren zur Beschichtung verwendet, so ist die maximal erreichbare Schichtdicke aufgrund der Wirtschaftlichkeit auf einige 10 µm begrenzt. Eine Vereinzelung der Stifte ist bei diesem Verfahren ebenfalls notwendig. Eventuell müssen die Stifte für das Auftragen maskiert werden, so dass das Verfahren für das Aufbringen einer ausreichenden Lotmenge nicht rentabel ist.

Auch auf der Implantatinnenseite an der Kontaktstelle zwischen Stift und Platine, welche normalerweise mittels einer Weichlotverbindung im SMT-Verfahren gefügt wird, erfolgt die Herstellung zunächst separat. Durch Hinzufügen weiterer Komponenten während oder nach dem Hochtemperaturlötprozesses wird die geforderte Weichlötbarkeit für den SMT-Prozess hergestellt.

Insgesamt ist eine Verarbeitung von Schüttgut in der Produktion problematisch, da die Bauteile vor dem Prozessieren jeweils vereinzelt, ausgerichtet und gegebenenfalls orientiert werden müssen, was einen zusätzlichen Prozessschritt darstellt, der für Mess- oder Prüfaufgaben in einer Teil- beziehungsweise vollautomatisierten Produktionsanlage erforderlich ist.

Die Aufgabe der vorliegenden Erfindung besteht somit darin, die Herstellung der Durchführung beziehungsweise eines elektromedizinischen Implantats zu vereinfachen und kostengünstiger zu gestalten.

Die obige Aufgabe wird gelöst durch ein Verfahren zur Herstellung eines Stifts für die Durchführung eines elektromedizinischen Implantats mit den folgenden Schritten:
- Erstellen eines folien-, blech- oder bandförmigen Halbzeugs durch Zusammenfügen mindestens eines ersten Schichtelements enthaltend ein elektrisch leitendes, vorzugsweise biokompatibles, Material in Folien-, Blech- oder Bandform und mindestens eines zweiten Schichtelements enthaltend ein Lot und/oder ein gut weichlötbares Material, vorzugsweise in Draht-, Blech- oder Bandform, oder durch Aufbringen des mindestens einen zweiten Schichtelements auf das mindestens eine erste Schichtelement
- Aufbringen und gegebenenfalls Fügen mindestens eines Opfer-, Sekundär- oder Lötinhibitor-Schichtelements (3, 4) in Draht-, Blech- oder Bandform auf das Halbzeug; und
- zumindest teilweises Abtrennen eines Stifts oder eines Satzes mehrerer, mit einem Verbindungssteg verbundener Stifte von dem Halbzeug.

Das erfindungsgemäße Verfahren beruht auf der Erkenntnis der Erfinder, dass der Herstellungsprozess des Stifts grundsätzlich anders gestaltet werden muss, um wesentliche Vereinfachungen oder Kosteneinsparungen bei der Herstellung der Durchführung beziehungsweise des Implantats zu erzielen. Der Herstellungsprozess wurde nun derart verändert, dass zunächst die Elemente des Stifts in Folien-, Blech oder Bandform gefügt werden. Die Vereinzelung der Stifte erfolgt anschließend durch Abtrennen von der Folie, dem Blech oder Band.

Der Vorteil des erfindungsgemäßen Verfahrens besteht insbesondere darin, dass Schüttgut-Prozesse oder einzelne manuelle Montageprozesse von Kleinteilen vermieden werden, wenn die Trennung der Stifte oder der Stiftsätze später in der Prozesskette erfolgt, z.B. erst nach der Hochtemperaturverlötung. Mit dem erfindungsgemäßen Verfahren können zudem die Anteile des elektrisch leitenden Materials und des Hochtemperaturlotes bzw. weichlötbaren Materials gezielt in engen Grenzen eingestellt werden. Das erfindungsgemäße Verfahren erhöht zwar aufgrund des Fügens auf Rohmaterialebene den Materialeinsatz, kann aber aufgrund des vergleichsweise einfacheren und sicheren Fügeprozesses sowie der einfacheren Einbringung des Stifts in die Durchführung und einfacheren Verlöten die Qualität deutlich erhöhen und damit nachhaltig zur Senkung der Ausschusszahlen beitragen. Die Bestückungszeit sinkt bei SMT (Surface Mounting Technology) lötbaren Durchführungen und zehn Signalpins um etwa dreißig Prozent je Durchführung.

Viele Fehlerbilder, welche Aufgrund eines manuellen Bestückungsprozesses der einzelnen Komponenten zueinander in Erscheinung treten, können vermieden werden (z.B. "Koaxialität", "Kontaktelement fehlt"). Die Prüfung auf solche Fehlerbilder vereinfacht sich daher in der Serie erheblich.

Durch einen gemeinsamen Trennprozess aus vorher gefügten Materialien vereinfacht sich in der Konstruktionsphase das Abstimmen der Bauteiltoleranzen der Durchführungskomponenten aufeinander, da einige Teile bereits mit gleichen Toleranzen hergestellt werden.

Zudem vereinfacht sich die visuelle Prüfung und die maßliche Prüfung wird ebenfalls einfacher, da sie zumindest teilweise bereits an dem Halbzeug durchgeführt werden kann. So können die vorher noch sehr komplexen und punktuell auftretenden Prüfaufgaben, auf mehrere unabhängige Einzelprüfungen zerlegt werden. Durch das Fügen der Funktionselemente des Stiftes in einem separaten Prozess, wird das Auftreten von Fehlern im Fügeprozess zwischen Stift in Durchführung minimiert. Durch das Aussortieren von fehlerhaften Stiften bereits nach der Herstellung der Stifte kann verhindert werden, dass diese zu Durchführungen prozessiert werden. Dadurch wird die Wirtschaftlichkeit der Fügeprozesse erhöht.

Insgesamt werden durch das erfindungsgemäße Vorgehen, bei dem die verschiedenen Stiftmaterialien bereits im Halbzeugstadium miteinander gefügt werden, mehrere kostenintensive manuelle Einzelprozesse zu wenigen, gut beherrschbaren Prozessschritten zusammengeführt, die leicht automatisierbar sind. Durch die Umgestaltung der Prozesse können die Stifte sowohl als Band in mittleren Stückzahlen als auch im Rolle-zu-Rolle Verfahren in großen Stückzahlen kostengünstig gefertigt werden.

In einem bevorzugten Ausführungsbeispiel wird vor dem vollständigen Abtrennen des Stifts oder eines Satzes mehrerer Stifte von dem Halbzeug zunächst lediglich ein Teil der Kontur der Stifte aus dem Halbzeug abgetrennt (z.B. mittels Stanzen freigeschnitten) und anschließend mindestens ein Teilbereich des Stifts umgeformt (z.B. mittels Prägen, Biegen). Hierdurch kann das Umformen besonders einfach bewerkstelligt werden, da der Stift noch am Halbzeug befestigt ist. Einzelheiten zu den Umformprozessen werden weiter unten im Detail erläutert. Das vollständige Abtrennen des Stifts von dem Halbzeug erfolgt nach dem Umformen, beispielsweise mittels Stanzen oder entlang einer Sollbruchstelle.

Vorzugsweise werden bei der Herstellung des folien-, blech- oder bandförmigen Halbzeugs das erste Schichtelement und das zweite Schichtelement form- oder stoffschlüssig miteinander verbunden. Das Fügen kann dabei unter Wärmebeaufschlagung geschehen, wobei die Diffusion der Materialien der beiden Schichtelemente ineinander durch die verwendeten Materialien, die Schichtgeometrie und verwendeten Prozessparameter (zum Beispiel Diffusionsheizen) beeinflusst werden kann.

Alternativ kann das mindestens eine zweite Schichtelement mit dem ersten Schichtelement stoffschlüssig durch ein vakuumtechnisches Beschichtungsverfahren (PVD oder CVD) oder eine galvanische Beschichtung auf das erste Schichtelement aufgetragen und mit diesem verbunden werden. Vakuumtechnische Beschichtungsverfahren erzeugen Beschichtungsdicken zwischen 0,1 µm bis 10 µm, mittels galvanischer Beschichtung können ebenfalls typische Schichtdicken im Bereich von 0,1 µm bis 10 µm erzielt werden.

Vorzugsweise besteht das mindestens eine erste Schichtelement aus einem biokompatiblen elektrisch leitenden Material in Folien-, Blech- oder Bandform, enthält zum Beispiel ein Element der Gruppe Nb, Pt, Pd, Ta, Zr, Ir, Ru und Hf oder einer Legierung daraus, vorzugsweise enthält das mindestens eine erste Schichtelement PtIr10, PtRu10 und/oder medizinischen Edelstahl (zum Beispiel 316L). Weiter kann das mindestens eine erste Schichtelement mindestens ein Element der Gruppe Mo, Wo, Cr, V und A1 enthalten oder eine Legierung daraus, beispielsweise FeNi, FeNiCo, FeCr.

Vorzugsweise wird das mindestens eine erste Schichtelement vor dem Fügen mit dem mindestens einen zweiten Schichtelement gereinigt und entfettet. Weiter vorzugsweise kann es sich bei dem ersten Schichtelement um eine Folie oder ein Blech mit mindestens einer Stufe und/oder Nut und/oder mit mehreren Materialschichten (Mehrlagenblech) handeln.

Nach dem Herstellen des Halbzeuges kann dieses vermessen, nachbearbeitet und gegebenenfalls aussortiert werden. Weiter können Zwischenschritte wie Reinigen, Beizen, Polieren oder dergleichen jederzeit durchgeführt werden.

Das mindestens eine zweite Schichtelement enthält beispielsweise ein Hochtemperaturlot (zum Beispiel mindestens ein Element der Gruppe Au und Ag oder eine Legierung daraus (zum Beispiel AuNi, AuPt10, AgCu, AuCu) und/oder ein Aktivlot (zum Beispiel AuCuNi, Ti70Ni15Cu15, Ag68Cu26Ti6, Ti67Ni33) und/oder ein Glaslot, welches vorzugweise biokompatibel ausgeführt ist. Als Glaslote eignen sich Gläser mit herabgesetzten Erweichpunkt und definierter Zusammensetzung.

Als weichlötbare Materialien kann das zweite Schichtelement z. B. Cu, Ag, Au, Ni, Pd, Pt, Ir, Fe oder Legierungen insbesondere CuAg0.10, CuAg.10P, CuTeP enthalten. Alternativ kann das zweite Schichtelement als Schichtsystem aus diesen Materialien verwendet werden.

In einem bevorzugten Ausführungsbeispiel können für das Fügen des mindestens einen ersten Schichtelements mit dem mindestens einen zweiten Schichtelements die grundsätzlich bekannten Verfahren wie Plattieren, Walzen, Löten oder Rollnahtschweißen verwendet werden.

Das erfindungsgemäße Abtrennen eines Stifts oder eines Satzes mehrerer, mit einem Verbindungssteg verbundener Stifte von dem Halbzeug oder dem Rohling erfolgt vorzugsweise mittels Feinschneiden, Stanzen, Chemical Milling, Laserschneiden oder Wasserstrahlschneiden. Das jeweils verwendete Verfahren richtet sich nach der Geometrie und den Toleranzanforderungen für den jeweiligen Stift beziehungsweise Satzes mehrerer Stifte.

Es ist von Vorteil auf Teilbereiche oder Bereiche des Halbzeuges Schichten als Lötinhibitor-Schichten oder Sekundärschichten zu gestalten.
Beispielsweise wird auf das mindestens eine erste Schichtelement eine dünne Schicht (Schichtdicke mindestens 10 µm, vorzugsweise mindestens 50 µm) enthaltend mindestens ein Element der Gruppe Al, Mg, Ca, Zr, und Y oder eine Legierung aus diesen Elementen aufgebracht werden, welche vorzugsweise auf der Oberfläche des mindestens einen ersten Schichtelements zumindest teilweise neben dem mindestens einen zweiten Schichtelement mit dem Lot aufgebracht und gegebenenfalls mit diesem gefügt wird. Hierfür können die oben genannten Fügeverfahren verwendet werden. Vorzugsweise können die genannten Schichten sowohl selektiv als auch flächig oxidiert werden. Nicht zu beschichtende Stellen werden mit dabei mittels Photoresist oder Lack maskiert. Maskierte Bereiche bleiben vor der Oxidation geschützt und können so besser mit Lot benetzt werden, als die Oxide. Besonders vorteilhaft ist eine Oxidation während einer nasschemischen Behandlung (z.B Reinigung der Stifte), zu einer Metalloxid-Schicht, z.B. eine Aluminiumoxid-Schicht, eine Yttriumoxid-Schicht oder eine Zirkoniumoxid-Schicht, welche die Funktion einer Lötinhibitor-Schicht bzw. Lotbarriere übernimmt, stattfindet. Nach der Oxidation kann der Fotolack vom Bauteil entfernt werden und die normale chemische Behandlung durchgeführt werden.

Es ist weiter vorteilhaft, wenn das Halbzeug zusätzlich mindestens ein drittes Schichtelement enthaltend einen Isolator oder ein duktiles Metall aufweist, das mit dem mindestens einen ersten Schichtelement und/oder dem mindestens einen zweiten Schichtelement zusammengefügt ist. Vorzugsweise bildet das mindestens eine erste Schichtelement enthaltend einen metallischen Leiter und das mindestens eine dritte Schichtelement enthaltend einen Isolator ein Grundmaterial, das als Mehrlagenblech ausgebildet ist. Ein Stift mit einem derartigen Grundmaterial kann als mehrpoliger Durchführungs-Stift eingesetzt werden. Vorzugsweise wird das mindestens eine erste Schichtelement und/oder das mindestens eine zweite Schichtelement in Draht-, Blech- oder Bandform mit dem mindestens einen dritten Schichtelement in Draht-, Blech- oder Bandform mittels der oben beschriebenen Fügeverfahren zusammengefügt. Das duktile Metall gewährleistet eine bessere Umformbarkeit beim Umformen (z.B. Rundprägen) und verhindert das seitliche Wegfließen des Lots des zweiten Schichtelements.
Insbesondere beim Stanzen oder Prägen ist es von Vorteil, wenn die Flächen eben sind. Hierzu können auf das mindestens eine erste Schichtelement eine Opferschicht aus einem duktilen Material (z.B. Aluminium) aufgebracht werden, welche dazu dient Hohlräume zu füllen und die Höhe zwischen dem obersten erste Schichtelement und den obersten zweiten Schichtelement auszugleichen. Nach dem Umformen kann die Opferschicht in einem Reinigungsprozess mit einer Lauge sehr einfach von den anderen Schichtelementen entfernt werden. Vorzugsweise verwendet man Natronlauge mit einer Konzentration von 10-45% bei einer Temperatur von 30 bis 70°C.

In einem weiteren bevorzugten Ausführungsbeispiel wird eine Deckschicht zur verbesserten Weichlötbarkeit auf das Halbzeug oder als Schutzschicht, vorzugsweise mittels eines Galvanikbads, vor und/oder nach dem gegebenenfalls zumindest teilweisen Abtrennen des Stifts oder des Satzes mehrerer Stifte von dem Halbzeug aufgebracht.

Die Deckschicht zur verbesserten Weichlötbarkeit wird vorzugsweise zumindest teilweise in dem Bereich vorgesehen, in dem das mindestens eine zweite Schichtelement enthaltend ein weichlötbares Material angeordnet ist. Hierdurch kann beispielsweise bei der Verwendung von Nickel als weichlötbares Material sichergestellt werden, dass keine offenen Nickelflächen am Stift existieren, aus denen während weiteren Verarbeitungsschritten unkontrolliert Nickel verschleppt werden kann und das weichlötbare Material durch ein Edelmetall (z.B. Au) gekapselt wird. Ferner kann durch eine erneute Beschichtung mit dem zweiten Schichtelement bzw. einem Legierungsbestandteil nach einem Prozessschrittschritt, die Schichtdicke homogenisiert werden. So kann gewährleistet werden, dass der Stift sich beim Löten auf die Platine oder in die Ausnehmung der Durchführung von allen Seiten, das heißt auch an den vom Halbzeug abgetrennten Kanten gleichmäßig mit dem Weichlot benetzen lässt. Eine derartige Deckschicht kann beispielsweise aus Au oder Pd oder eine Legierung aus anderen Edelmetallen bestehen. In Abhängigkeit von anzubindenden Material und Herstellungsprozess sind unterschiedliche Oberflächenveredelungen (zum Beispiel ENIG - Electroless Nickel Immersion Gold -, ENEPIG - Electroless Nickel Electroless Palladium Immersion Gold oder HAL Gold - Hot Air Leveled Gold) möglich.

Eine Schutzschicht kann auf die Oberfläche des ersten und/oder zweiten Schichtelements bis zur weiteren Verarbeitung als Versiegelung aufgetragen werden, wobei hierfür ein Polymer oder ein organischer Schutzfilm verwendet wird (OSP- Organic Surface Protection). Bekannte Schutzfilme können vollständig oder selektiv auf den Stift nach dem teilweisen oder vollständigen Abtrennen abgeschieden werden. Typische Schichtdicken betragen 0,2 µm bis 0,6 µm und enthalten zum Beispiel substituierte Imidiazole und/oder Triazole. Der Schutzfilm verhindert die Oxidation des Grundmaterials während der Lagerung typischerweise für mehrere Monate und pyrolysiert unmittelbar vor bzw. während des Hartlöt-bzw. Weichlötprozesses. Der Schutzfilm wird mittels Beschichten (z.B. Lackieren, Tauchen) auf die Stiftabschnitte oder im Rolle-zu-Rolle Verfahren unmittelbar nach dem Reinigen oder Beizen aufgebracht.

Als besonders vorteilhaft hat sich erwiesen, dass in einem Ausführungsbeispiel des erfindungsgemäßen Verfahrens der Stift vor oder nach dem zumindest teilweisen Abtrennen von dem Halbzeug umgeformt wird, vorzugsweise derart, dass das mindestens eine zweite Schichtelement den Stift mindestens teilweise umgibt. Dies ist deshalb von Vorteil, weil beim Abtrennen des Stifts beziehungsweises des Satzes mehrerer Stifte aus dem Halbzeug eine scharfe Trennkante entsteht und beispielsweise das Lotdepot nicht oder nur teilweise auf der Schnittfläche angeordnet ist. Beim Verlöten des Stifts mit der Durchführung verteilt sich das Lot um den Stift. Um eine homogenere Verteilung des Lots um den Stift zu erreichen, ist es deshalb vorteilhaft, das Lotdepot mittels Umformen über alle Seiten des Stifts zu verteilen beziehungsweise zu verschmieren. Durch das Verteilen wird sichergestellt, dass sich der Lotkegel beim Aufschmelzen um den Stift schließt und somit eine sichere Verbindung herstellt. Die Wahrscheinlichkeit von Fehlern im Lot wird daher minimiert. Gleiches gilt analog auch für das mindestens eine zweite Schichtelement enthaltende eine weichlötbares Material.

Es ist weiter von Vorteil, dass der Stift oder jeder Stift des Satzes mehrerer Stifte nach dem zumindest teilweisen Abtrennen derart umgeformt wird, dass mindestens ein vorzugsweise rundumlaufender Vorsprung und/oder mindestens eine Ausnehmung entsteht, wobei der Vorsprung und/oder die Ausnehmung jeweils an der Seitenfläche (Mantelfläche) des jeweiligen Stifts angeordnet ist. Mit einem derartigen Vorsprung oder Anschlag kann sich der Stift beim Lötprozess in der Durchführung selbst in Position halten oder es kann eine korrekte Anordnung des Stifts in der Durchführung erreicht werden. Eine Ausnehmung kann zusätzlich als Lötstopp dienen.

Es ist weiter von Vorteil, wenn das zumindest teilweise Abtrennen in eine Richtung im Wesentlichen senkrecht (quer) zur Richtung des Zusammenfügens des mindestens einen ersten Schichtelements mit dem mindestens einen zweiten Schichtelement und/oder dem mindestens einen dritten Schichtelement erfolgt. Hierdurch ist beispielsweise die Walzrichtung, die Plattierrichtung, das heißt die Fügerichtung der Materialien, im Wesentlichen senkrecht zur Stiftlängs- und Stanzrichtung, so dass Gefügefehler oder Leckpfade vermieden werden.

Weiter ist von Vorteil, wenn ein Abschnitt des Stifts nach dem zumindest teilweisen Abtrennen rund geformt wird, insbesondere der sich entlang der Längsrichtung des Stifts erstreckende Bereich des Schafts, welcher in der Durchführung verbaut wird und mit welchem der Stift in die Durchführung eingesteckt wird. Dies ist deshalb von Vorteil, weil die durchgehenden Ausnehmungen in den Durchführungs-Keramiken häufig rund beziehungsweise kreisförmig ausgeführt werden. Dies führt zu einer gleichmäßigeren Hartlotverteilung während der flüssigen Phase des Lotes im Spalt sowie zu einer Verringerung des benötigten Hartlotes. Das Rundprägen kann zum Beispiel mittels eines Verbundfolgeschneidwerkzeugs realisiert werden. Vorteilhaft ist das Prägewerkzeug im Einflussbereich des Lotes überwiegend senkrecht zur Stiftachse zu strukturieren, so dass definierte Mikrorauhigkeit von mindestens 2µm entsteht. Durch das Abformen der Oberflächenstruktur der Prägestempel können Lötinhibitorschichten aus den obersten Schichtelementen in den Stift geprägt werden, welche sich durch ihre Mikrorauheit und Oberflächenstruktur von den darunterliegenden Schichten unterscheiden.

In einem weiteren bevorzugten Ausführungsbeispiel wird der Stift nach dem zumindest teilweisen Abtrennen an mindestens einem Endabschnitt in eine Gullwing, J-Lead oder SOP-ähnliche Form umgeformt. Andere Formen aus der Mikroelektronik sind ebenfalls denkbar. Hierdurch kann die Kontaktfläche zur Platine bzw. die anhaftende Weichlotmenge erhöht und die erforderliche Abzugskraft zwischen Platine und Durchführung vergrößert werden.

Die Effizienz während der Herstellung einer Durchführung kann weiter dadurch erhöht werden, dass zunächst lediglich ein Satz mehrerer, mit einem Verbindungssteg verbundener Stifte von dem Halbzeug abgetrennt wird, wobei vorzugsweise die Stifte des Satzes teilweise voneinander abgetrennt sind. Die Stifte dieses Satzes können anschließend gemeinsam, wie oben beschrieben beschichtet und/oder umgeformt werden und gemeinsam mittels Hochtemperaturlöten in der Durchführung befestigt werden, wobei jeder Stift in einer separaten Ausnehmung angeordnet wird. Der Verbindungssteg schützt die Stifte während des Transportes vor Deformation und gewährleistet eine gleichmäßige Ebenheit während der Montage über alle Stifte des Verbindungssteges hinweg. Nach dem Fügen in der Baugruppe werden die Stifte vorzugsweise durch Entfernung des Verbindungsstegs voneinander getrennt. Hierfür kann in einem bevorzugten Ausführungsbeispiel des erfindungsgemäßen Verfahrens in einem Endabschnitt eines Stifts, vorzugsweise neben dem Verbindungssteg, eine Sollbruchstelle eingebracht werden. Dies kann durch Einbringen einer Einkerbung oder einer durchgehenden Ausnehmung in diesem Bereich realisiert werden. Alternativ können die gemeinsam montierten Stifte durch einen einzelnen Beschneidungsprozess auf gleicher Höhe gekürzt werden. Hierbei wird die Durchführung am Flansch sowie am Verbindungssteg geklemmt und anschließend über alle Stifte gleichzeitig abgetrennt.

Die obige Aufgabe wird ferner durch ein Verfahren zur Herstellung einer Durchführung für ein elektromedizinisches Implantat gelöst, wobei ein Körper einer Durchführung mit mindestens einer durchgehenden Ausnehmung bereitgestellt wird, weiter die oben angegebenen Schritte zur Herstellung eines Stifts erledigt werden und der zusätzliche Schritt durchgeführt wird, dass der Stift oder jeder Stift des Satzes mehrerer Stifte mit der Innenfläche einer durchgehenden Ausnehmung des Körpers mittels Löten verbunden wird. Dieses erfindungsgemäße Verfahren weist die oben angegebenen Vorteile gegenüber den herkömmlichen Verfahren auf. Besonders bevorzugt werden die mehreren Stifte des Satzes anschließend voneinander getrennt, vorzugsweise durch Entfernen eines Verbindungsstegs und/oder entlang einer zuvor in den jeweiligen Stift eingebrachten Sollbruchstelle oder einer gemeinsamen Flucht.

Die obige Aufgabe wird ferner durch ein Verfahren zur Herstellung eines elektromedizinischen Implantats mit einer Platine und mit den oben angegebenen Schritten zur Herstellung einer Durchführung gelöst, wobei anschließend jeder Stift der Durchführung mit einem Anschluss der Platine, vorzugsweise mittels Löten oder Schweißen, verbunden wird. Danach wird die Platine in einem Gehäuse des Implantats angeordnet und die Durchführung hermetisch dicht mit dem Gehäuse verbunden.

Die obige Aufgabe wird auch gelöst durch einen Stift für ein elektromedizinisches Implantat hergestellt oder herstellbar mit einem oben angegebenen Verfahren.

Die Aufgabe wird ferner gelöst durch eine Durchführung für ein elektromedizinisches Implantat hergestellt oder herstellbar mit einem oben dargestellten Verfahren.

Die Aufgabe wird ferner gelöst durch ein elektromedizinisches Implantat hergestellt oder herstellbar mit einem oben angegebenen Verfahren.

Das erfindungsgemäße Verfahren zur Herstellung eines Stifts beziehungsweise einer Durchführung beziehungsweise eines elektromedizinischen Implantats sowie derart hergestellte Stifte beziehungsweise Durchführungen werden nachfolgend anhand von Figuren erläutert. Dabei bilden alle abgebildeten und/beschriebenen Merkmale den Gegenstand der Erfindung, auch unabhängig von ihrer Zusammenfassung in den Ansprüchen oder deren Rückbeziehung.

Es zeigen schematisch:
- Fig. 1: einen Querschnitt durch ein Halbzeug in Form eines Bandes für einen erfindungsgemäßen Stift eines ersten Ausführungsbeispiels,
- Fig. 2: einen erfindungsgemäßen Stift eines zweiten Ausführungsbeispiels nach der Abtrennung von dem Halbzeug in einer Ansicht von oben,
- Fig. 3: ein weiteres Ausführungsbeispiel eines erfindungsgemäßen Stifts nach der Abtrennung von dem Halbzeug in einer perspektivischen Ansicht,
- Fig. 4 bis 17: weitere Querschnitte durch Halbzeuge in Form eines Bandes für weitere Ausführungsbeispiele erfindungsgemäßer Stifte,
- Fig. 18: ein weiteres Ausführungsbeispiel eines erfindungsgemäßen Stifts in einer perspektivischen Ansicht von der Seite,
- Fig. 19 und 20: einen Endabschnitt eines weiteren Ausführungsbeispiels eines erfindungsgemäßen Stifts in einer Ansicht von der Seite vor dem Verlöten mit dem Anschluss einer Platine (Fig. 19) und nach dem Verlöten (Fig. 20),
- Fig. 21 bis 24: Endabschnitte weiterer Ausführungsbeispiele eines erfindungsgemäßen Stifts in einer Ansicht von der Seite vor dem Verlöten mit dem Anschluss einer Platine (Fig. 19 und 21) und nach dem Verlöten (Fig. 22 und 24),
- Fig. 25 bis 47: weitere Ausführungsbeispiele erfindungsgemäßer Stifte jeweils in einer Ansicht von der Seite,
- Fig. 48 bis 51: Abschnitte weiterer Ausführungsbeispiele erfindungsgemäßer Stifte in einer Ansicht von der Seite,
- Fig. 52: einen Abschnitt eines Halbzeugs zur Herstellung erfindungsgemäßer Stifte, welcher verschiedene Schritte der Herstellung eines Stifts in einer Ansicht von oben veranschaulicht,
- Fig. 53 bis 55: drei Ausführungsbeispiele erfindungsgemäßer Durchführungen jeweils in einer Ansicht von der Seite,
- Fig. 56: ein weiteres Ausführungsbeispiel einer erfindungsgemäßen Durchführung in einer perspektivischen Ansicht von der Seite,
- Fig. 57: das Ausführungsbeispiel einer erfindungsgemäßen Durchführung gemäß Fig. 56 in einer Ansicht von oben,
- Fig. 58: das Ausführungsbeispiel einer erfindungsgemäßen Durchführung gemäß Fig. 56 in einer Ansicht von der Seite,
- Fig. 59: das Ausführungsbeispiel einer erfindungsgemäßen Durchführung gemäß Fig. 56 in einer weiteren Ansicht von der Seite und
- Fig. 60: einen Abschnitt eines Halbzeugs zur Herstellung erfindungsgemäßer Stifte, welcher verschiedene Schritte (A bis F) der Herstellung eines Stifts in einer Ansicht von oben veranschaulicht.

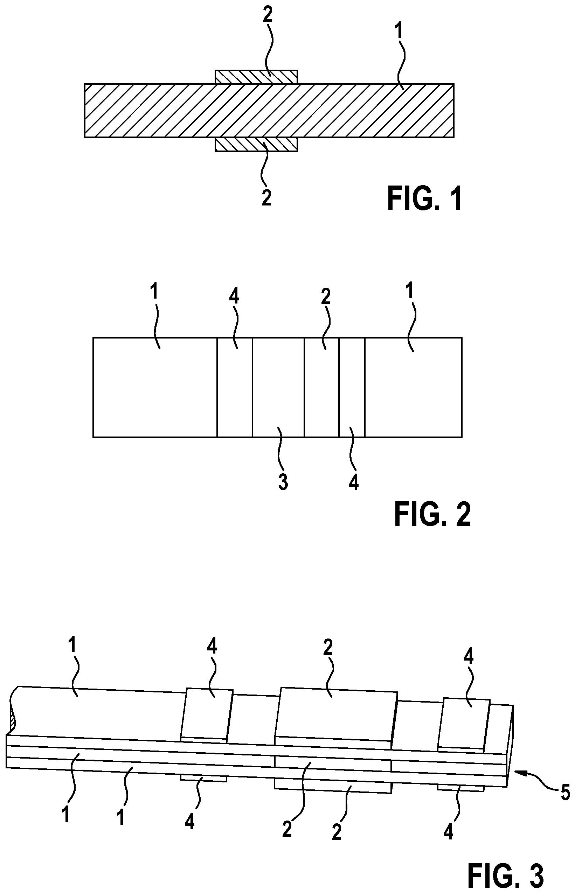

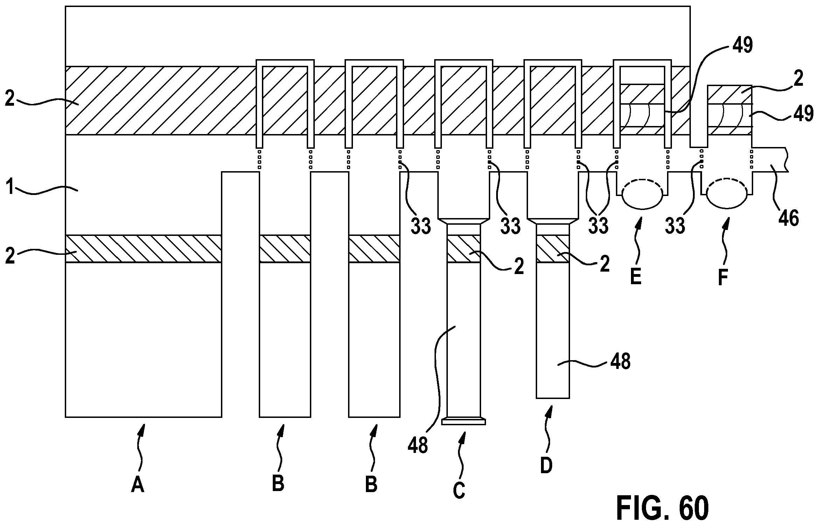

In einem Ausführungsbeispiel des erfindungsgemäßen Verfahrens wird ein erstes Schichtelement 1 in Form eines Bleches oder Bandes aus einem biokompatiblen elektrisch leitenden Material (zum Beispiel Niob) zunächst mit einem Lösemittel (z.B. Aceton) gereinigt und entfettet. Auf mindestens einer Seite des ersten Schichtelements 1 wird ein zweites Schichtelement 2 aus einem Lot (z.B. Au-Lot) oder einem weichlötbaren Material (z.B. Nickel) aufgebracht. Das zweite Schichtelement (Lotschicht) 2 wird insbesondere formoder stoffschlüssig mit dem ersten Schichtelement verbunden, hierfür ist es von Vorteil wenn sich im ersten Schichtelement ein Anschlag oder eine Nut befindet, in der das zweite Schichtelement formschlüssig eingepasst und relativ auf dem ersten Schichtelement positioniert werden kann. Die Breite der Nut muss dabei mindestens so groß sein wie das zweite Schichtelement sowie die durch die Wärmeausdehnung verursachte Längenausdehnung. Das zweite Schichtelement 2 wird auf das erste Schichtelement gefügt. Zum Beispiel mittels Löten. Dabei ist es von Vorteil, einen mehrstufigen Lötprozess zu verwenden, bei dem zuerst das Höherschmelzende zweite Schichtelement auf das das erste Schichtelement gefügt wird. Zum Beispiel wird zuerst das Nickel Schichtelement bei einer Temperatur von 1100-1380°C gefügt. Anschließend wird das zweite Schichtelement 2 aus Goldlot bei einer Temperatur von 950-1090°C angelötet. Alternativ können andere Fügeverfahren beispielsweise Plattieren, Warmpressschweißen, Kaltwalzplattieren oder Rollennahtschweißen verwendet werden. Nach jedem Fügeschritt findet eine eine Prüfung des Fügebereiches statt. Hierfür eignet sich zum Beispiel eine integrierte visuelle Inspektion, Röntgeninspektion oder Thermographie, um fehlerhafte Bandbereiche zu erkennen und automatisiert von der weiteren Verarbeitung auszuschließen. Die Breite des Bandes für das erste Schichtelement 1 beträgt vorzugsweise mindestens der Länge des herauszutrennenden Stiftes plus Seitenflächen, die zur Führung des Bandes dienen. Vorzugsweise sind die Führungsflächen des Bandes für das erste Schichtelement 1 mit Öffnungen versehen, um eine sehr genaue Positionierung des Bandes im Bereich von wenigen hundertstel Millimetern oder weniger zu ermöglichen. Um eine maschinelle Führung zu ermöglichen, ist es hilfreich Bohrungen, bzw. Passungen oder Ausnehmungen in den Seiten des Bandes bzw. Blechs welche als Zentrierungen oder Anschläge dienen können vorzusehen. Vorzugsweise ist das Band für das erste Schichtelement 1 noch breiter, und zwar so, dass eine ganze Anzahl an Stiften aus dem Band herausgetrennt werden kann. Die Dicke des Bandes für das erste Schichtelement 1 entspricht vorzugsweise der Dicke ersten Schichtelements 1, aus dem der Stift später besteht oder ist geringfügig dicker bzw. dünner, um Dickenänderungen durch das Walzen, Plattieren, bzw. Warmpressschweißen, Löten oder Rollennahtschweißen etc. auszugleichen. Vergleichbare Überlegungen gelten für das zweite Schichtelement 2 und weitere Schichtelemente, wobei die Bänder der weiteren Schichtelemente ohne Zusatz-Seitenflächen als Band oder Draht zugeführt und auf das erste Schichtelement 1 aufgetragen werden. Ein derartig gefertigtes Halbzeug ist in Figur 1 beziehungsweise in Abschnitt A der Figur 60 dargestellt, wobei bei dem in Figur 1 dargestellten Ausführungsbeispiel die Ober- und die Unterseite des ersten Schichtelements 1 jeweils mit einem zweiten Schichtelement 2 versehen sind. Die Lotschicht ist daher auf beiden Seiten des ersten Schichtelements 1 aufgebracht. Anschließend wird ein Stift beispielsweise mittels Stanzen, Chemical Milling, Laserschneiden oder Wasserstrahlschneiden von dem Halbzeug zumindest teilweise abgetrennt. Die Breite eines derartigen abgetrennten Abschnitts des Halbzeugs, wie er beispielsweise auch in den Figuren 2 und 3 oder in Abschnitt B der Figur 60 dargestellt ist, beträgt etwa 0,1 bis 2 mm und die Länge etwa 0,5 bis 50 mm.

In Figur 2 ist ein weiteres Ausführungsbeispiel eines von einem Halbzeug abgetrennten Stifts dargestellt, wobei auf dem ersten Schichtelement 1 zusätzlich noch der Einflussbereich bzw. Schmelzbereich des Lotes 3 (z.B. aus Niob) rechts neben dem zweiten Schichtelement 2, das vorzugsweise eine Lotschicht (z.B. aus Gold) darstellt, sowie links neben dem Haftschichtelement 3 und rechts neben dem zweiten Schichtelement 2 eine Antibenetzungsschichtelement 4 (Lötinhibitorschicht) (Zirkoniumoxid) aufgebracht sind. Im Einflussbereich des Lotes 3 wird die Oberfläche durch Walzen oder Prägen gezielt eingestellt, um die Haftung zwischen Lot und dem dritten Schichtelement zu verbessern. Vorteilhaft ist es die Rauheit im Bereich 3 definiert einzustellen. Insbesondere ist es vorteilhaft die Mikrorauheit im Loteinflussbereich 60°-120° senkrecht zur Stiftachse zu gestalten.
Die Lotschicht 2 besteht z. B. aus Hochtemperaturloten wie Au, AuAg8, AuPt10, Ti60Ni25Cu15. Das Antibenetzungsschichtelement 4 wirkt als Lötstopp und besteht z. B. aus Keramikschichten bzw. Keramik enthaltenden Schichten, wie z. B. Al203, ZrO2, TiO2, etc., oder Graphit bzw. Graphit enthaltende Schichten oder Metallen bzw. deren Legierungen, die für das Hartlot des zweiten Schichtelementes 2 nicht benetzend wirken. Nach der (Hochtemperatur-)Verlötung können die Antibenetzungsschichtelemente 4 entfernt werden, z. B. mittels Bürsten, Nassreinigen, chemisch Ätzen, etc.

Ein Antibenetzungsschichtelement 4 ist auch bei dem in Figur 3 gezeigten Ausführungsbeispiel vorgesehen, jedoch mit einem Abstand von jeder Seite des zweiten Schichtelements 2 (z.B. bestehend aus Au-Lot). Die Basisschicht mit dem ersten Schichtelement 1 ist bei diesem Ausführungsbeispiel aus drei übereinanderliegenden, elektrisch leitenden Schichten aufgebaut, wobei in dem Bereich, in dem außen auf dem elektrischen Leiter eine Lotschicht aufgebracht ist, auch im Inneren, zwischen zwei ersten Schichtelementen (elektrisch leitende Schichten) 1 ein zweites Schichtelement 2 in Form einer Lotschicht (z.B. Au-Lot) vorgesehen ist. Dies hat den Vorteil, dass auch an der vorn sichtbaren Trennkante 5 ein Lotmaterial angeordnet ist.

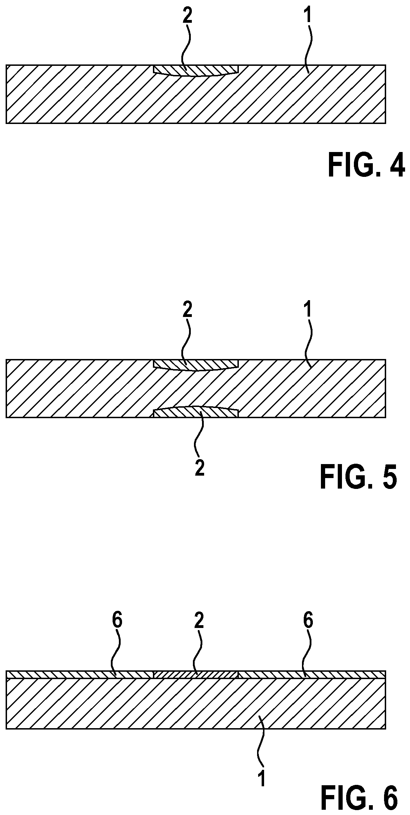

Die Figuren 4 und 5 zeigen Halbzeuge für erfindungsgemäße Stifte, welche die zweiten Schichtelemente 2 in einer Vertiefung des ersten Schichtelements 1 aufweisen, so dass das erste Schichtelement 1 mit der Oberseite der elektrisch leitenden Schicht bündig verläuft. Bei der Herstellung wird das erste Schichtelement aus mehreren Schichten gefertigt bzw. es wird eine Nut von etwa 80%-120% der Tiefe des zweiten Schichtelementes vorgesehen. Die Breite der Nut muss dabei mindestens so groß sein wie das zweite Schichtelement sowie die durch die Wärmeausdehnung verursachte Längenausdehnung. Das zweite Schichtelement 2 wird auf das erste Schichtelement gefügt. Zum Beispiel mittels Löten. Dabei benetzt das Lot die Seitenwände des ersten Schichtelements unter einem Meniskus. Durch Folgeprozesse wie Glattwalzen, Polieren, Schleifen, können Höhenunterschiede des Lotbereiches sowie Unebenheiten des Fügeprozesses ausgeglichen werden.

In Figur 6 wird ein Halbzeug dargestellt, bei dem das Material des ersten Schichtelements 1 mittels einer Deckschicht 6 in Form einer Polymerschicht oder einer OSP-Schicht gegenüber Oxidation versiegelt wird. Das Material der Deckschicht 6 wird auf die Oberfläche des ersten Schichtelements mittels Lackieren aufgetragen. Unmittelbar vor der Verarbeitung kann die Deckschicht 6 teilweise oder vollständig, beispielsweise mittels Lösemitteln (z.B. Aceton), entfernt werden. Alternativ pyrolisiert die OSP-Schicht und kann anschließend entfernt werden, z. B. mittels Bürsten, Nassreinigen, chemisch Ätzen, etc.

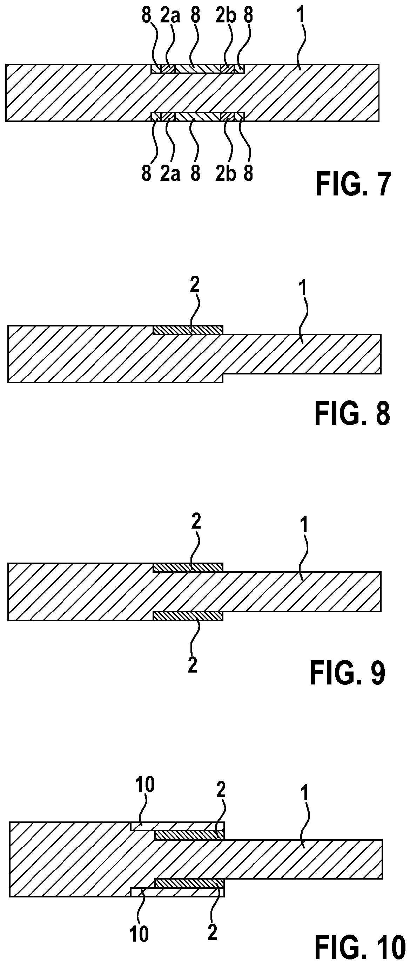

Figur 7 zeigt ein Halbzeug mit einem ersten Schichtelement und in einer Ausnehmung mit zwei zweiten Schichtelementen 2a, 2b, die Hochtemperaturlote verschiedener Zusammensetzung enthalten. Beispielsweise können die Hochtemperaturlote sich in ihrer Zusammensetzung und gegebenenfalls auch in ihrem Schmelzpunkt unterscheiden. Die Ausnehmung wird durch dritte Schichtelemente 8 aus duktilem Metall (z.B. Aluminium) ausgefüllt, welches eine bessere Umformbarkeit beim Umformen, z.B. beim Rundprägen, des Stiftes gewährleistet. Weiter verhindert das duktile Material während des Rundprägens ein seitliches Wegfließen des Materials des jeweiligen zweiten Schichtelements 2a, 2b. Dadurch wird sichergestellt, dass die Verteilung des jeweiligen zweiten Schichtelements 2a, 2b nach dem Umformen homogen und gleichmäßig um den Stift erfolgt.

In dem in der Figur 8 gezeigten Ausführungsbeispiel ist das zweite Schichtelement 2 an einer Kante des ersten Schichtelements 1 angeordnet, so dass dieses auf einer Seite mit der Oberfläche des ersten Schichtelements 1 bündig verläuft. Das erste Schichtelement 1 ist daher im Bereich seiner Oberfläche durch die Stufe profiliert.

In Figur 9 zeigt ein Halbzeug, bei dem das zweite Schichtelement 2 stoffschlüssig an eine Kante des ersten Schichtelementes 1 angefügt ist. Die Anordnung kann, wie dargestellt, auf maximal zwei Seiten des Halbzeugs erfolgen. Durch das Platzieren an der Kante erhält das Hochtemperaturlot des zweiten Schichtelements 2 beim Aufschmelzen eine Vorzugsrichtung. Das Lot wird sich überwiegend in der Ebene des jeweiligen zweiten Schichtelements 2 verteilen.

Bei dem in Figur 10 gezeigten Ausführungsbeispiel ist im Bereich des zweiten Schichtelements 2 und des angrenzenden ersten Schichtelements 1 eine Deckschicht 10 vorgesehen, welche mittels Fügen auf das erste Schichtelement 1 und das zweite Schichtelement 2 aufgebracht wird. Durch die Profilierung des ersten Schichtelements 1 und die Anordnung des zweiten Schichtelements 2 direkt an der Kante des ersten Schichtelements 1 sowie durch die Anordnung der Deckschicht 10 wird eine Vorzugsrichtung für das Schmelzen bzw. Herauslaufen des Lotes des zweiten Schichtelements 2 vorgegeben (von links nach rechts). Ein Zusammenziehen Aufgrund der Oberflächenspannung des Lotes wird durch die Deckschicht 10 verhindert. Das Lot wird durch die Deckschicht 10 bis zum Aufschmelzen vor Umwelteinflüssen (z.B. Oxidation, Beschädigung) geschützt.

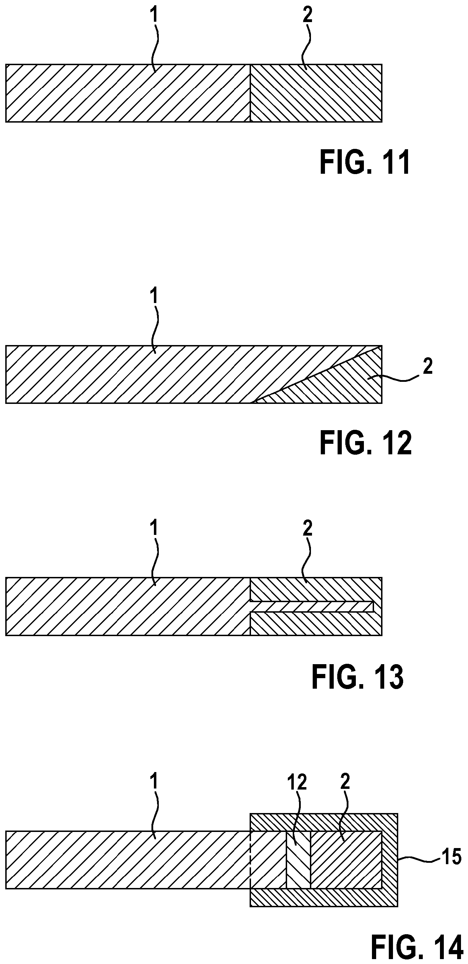

Figur 11 zeigt ein Ausführungsbeispiel, bei dem ein erstes Schichtelement 1 in Form einer biokompatiblen, elektrisch leitenden Schicht 1 mit einem daneben angeordneten zweiten Schichtelement 2 in Form einer Nickel-Schicht als weichlötbares Material verbunden und zusammengefügt ist. Somit ist ein Endabschnitt des Stifts nach dieser Ausführungsform als Nickel-Abschnitt ausgebildet und lässt sich daher mit einem Kontakt einer Platine gut verlöten. Der Querschnitt des zweiten Schichtelements ist, wie aus Figur 11 hervorgeht, rechteckig.

In den in Figuren 12 und 13 gezeigten Ausführungsbeispielen sind andere Formen des zweiten Schichtelements 2 gezeigt, wobei der Querschnitt in Figur 12 dreieckig und in Figur 13 U-förmig ist. Insbesondere mit dem dreieckigen Querschnitt wird ein stofflicher Übergang von dem elektrisch leitenden Material des ersten Schichtelements 1 zu dem weichlötbaren Material des zweiten Schichtelements 2 erreicht.

In Figur 14 ist dargestellt, dass durch entsprechende prozessuale Schritte, wie Ausheizen, zwischen dem ersten Schichtelement 1 und dem zweiten Schichtelement 2 eine Diffusionszone 12 ausgebildet werden kann, in der der Übergang der Materialien beider Schichtelemente 1,2 ineinander stattfindet.

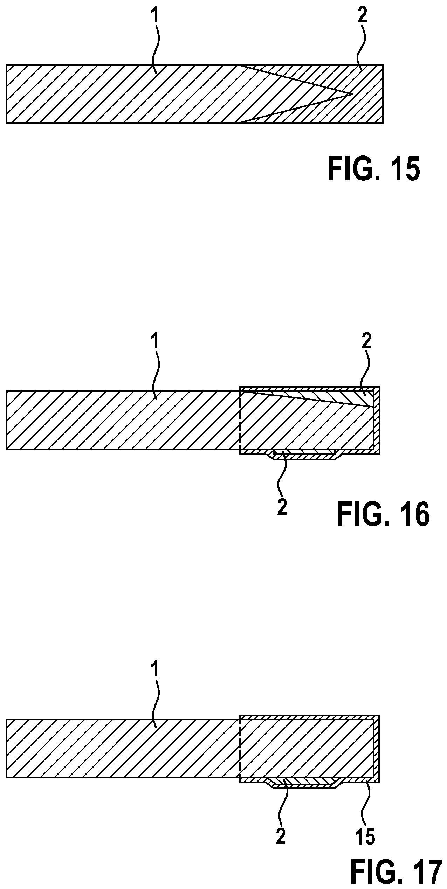

In den in Figuren 14, 16 und 17 dargestellten Ausführungsbeispielen ist zusätzlich eine Beschichtung 15 vorgesehen, welche einen Korrosionsschutz für das zweite Schichtelement 2, das häufig Ni aufweist, beispielsweise in Form einer Pd-Schicht, darstellt. Eine derartige Schicht kann beispielsweise mittels CVD oder PVD aufgebracht werden.

Für die Anordnung eines zweiten Schichtelements 2 in Form einer weichlötbaren Schicht sind auch die in den Figuren 1, 4, 5, 8, 9 und 10 gezeigten Anordnungsvarianten anstelle der Lotschicht denkbar.

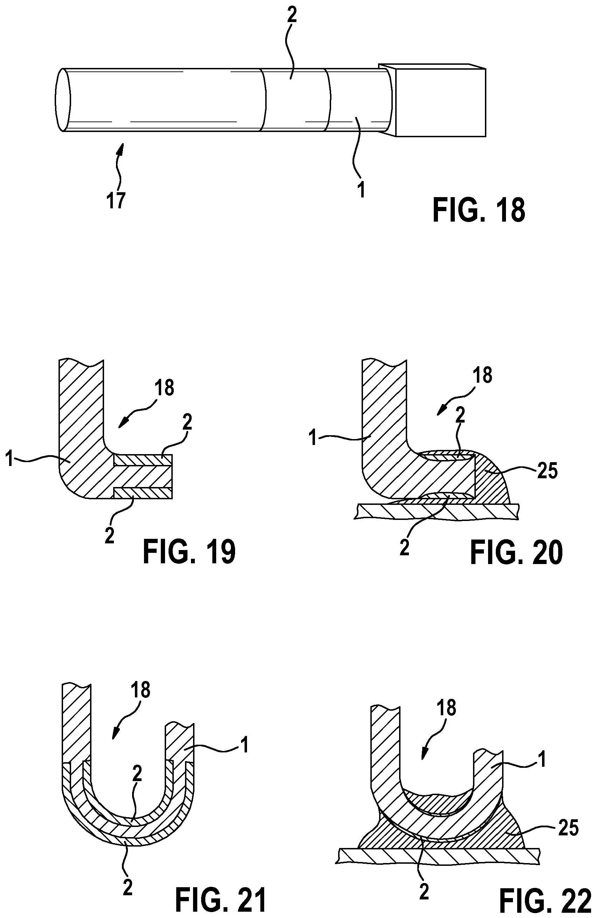

Figur 18 zeigt ein Ausführungsbeispiel eines erfindungsgemäßen Stifts, bei dem der Endabschnitt 17, mit dem der Stift durch den Isolator 20 der Durchführung hindurch gesteckt werden kann, nach dem Abtrennen vom Halbzeug rund geprägt wurde. Das zweite Schichtelement liegt im rundgeprägten Bereich, so dass sich das Lot möglichst gleichmäßig im Keramikelement verteilen kann. Der Bereich gegenüber dem rundgeprägten Endabschnitt 17 enthält noch die ursprüngliche Kontur des Ausgangsmaterials.

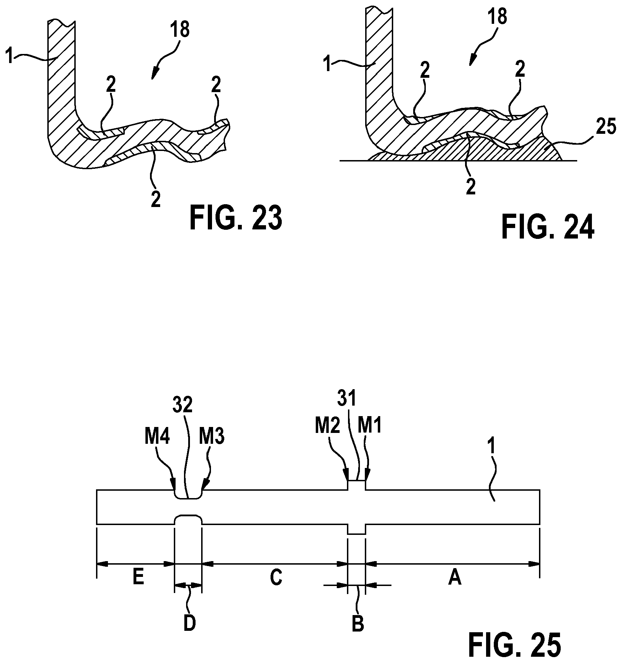

Die Figuren 19, 21 und 23 zeigen verschiedene Ausführungsbeispiele von erfindungsgemäßen Stiften, deren Endabschnitt 18, welcher mit einem Anschluss einer Platine verbunden werden soll, nach dem Abtrennen von dem Halbzeug in eine vorgegebene Form umgeformt wurde. Der in Figur 19 gezeigte Endabschnitt 18 weist eine Rundung in Form eines Viertelkreissegments ähnlich der sogenannten SOP-Form auf. In Figur 21 hat der Endabschnitt 18 eine sogenannte J-Lead-Form und in Figur 23 eine sogenannte Gullwing-Form. Die Anordnung des weichlötbaren Materials als zweites Schichtelement 2 erfolgt, wie aus den Figuren ersichtlich, zunächst wie oben beschrieben mittels Aufbringen auf ein Halbzeug und Abtrennen von diesem. Die endgültige Stiftgeometrie wird durch Umformen (z.B. Biegen, Stauchen) festgelegt. Die Position der gut weichlötbaren Materialen auf dem Stift kann durch das Fügen der Materialen sowie das Umformen beeinflusst werden. In den Figuren 20, 22 und 24 ist jeweils der Zustand, nach dem der jeweilige Stift mit seinem Endabschnitt 18 mit einem Anschluss bzw. Pad der Platine verlötet wurde, gezeigt. Die Platine ist nicht separat dargestellt. Es ist zu erkennen, dass das gut weichlötbare Material des zweiten Schichtelements 2 eine gute Benetzung und Haftvermittlung zwischen Stift und Platine bewirkt. Der Lotkegel 25 kann sich dabei über das zweite Schichtelement 2 hinaus aufbauen.

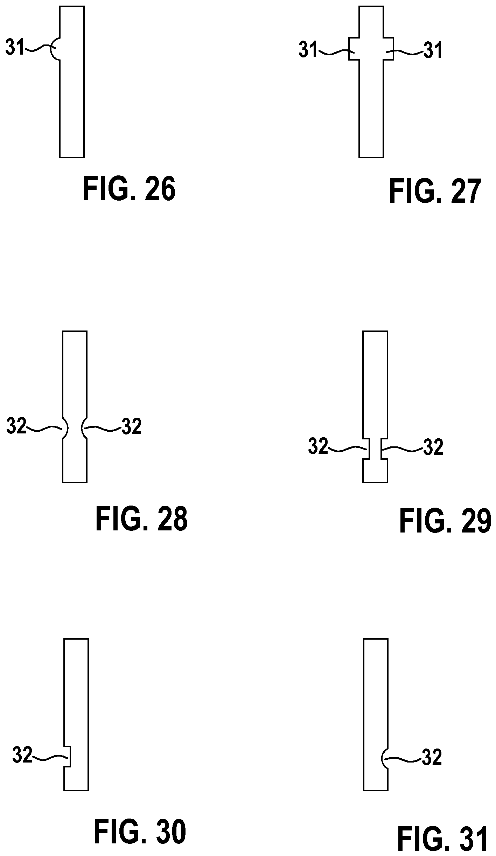

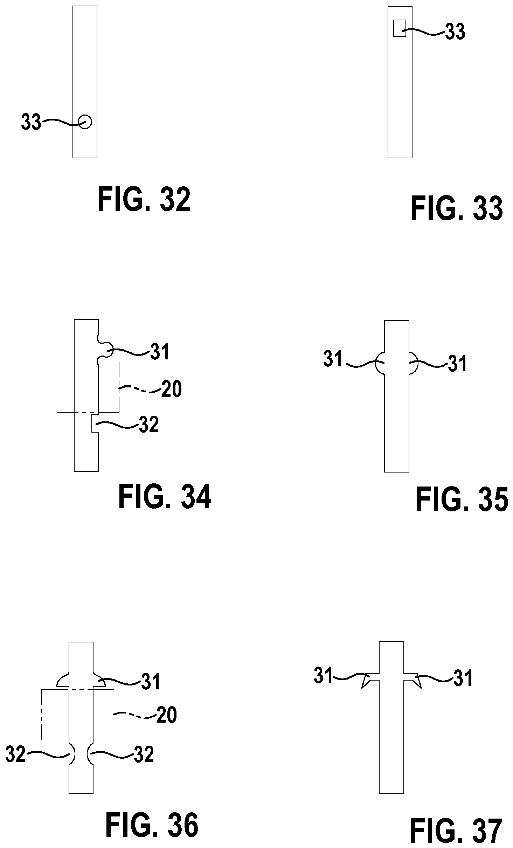

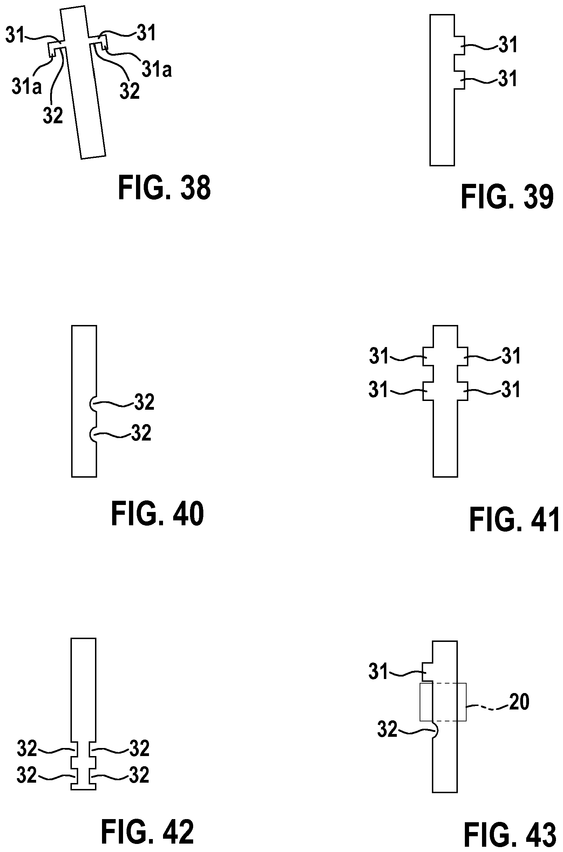

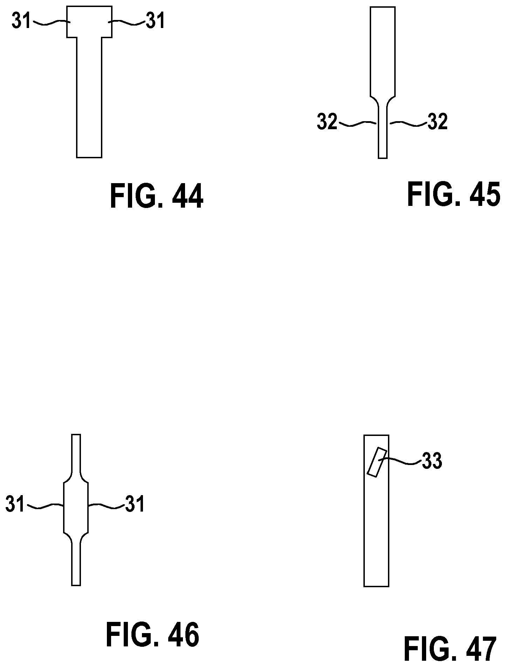

Es ist weiter von Vorteil, wenn das Halbzeug vor dem Abtrennen oder der Stift nach dem teilweisen oder vollständigen Abtrennen von dem Halbzeug umgeformt wird, so dass eine Vorsprung 31 und/oder eine Ausnehmung 32 entsteht. In den Figuren 25 bis 31, 34 bis 46 sind verschiedene Varianten derartiger Vorsprünge 31 oder Ausnehmungen (Aussparungen) 32 dargestellt. Sie können an verschiedenen Stellen an der Außenseite (Mantelfläche) des Stifts in Richtung der Längsachse vorgesehen sein. Hierbei wird durch einen Vorsprung 31 erreicht, dass der Stift während des Einlötens in die Durchführung, wie in den Figuren 34 und 36 gezeigt, als Anschlag wirkt und sich der Stift beim Lötprozess selbst in Position hält. Weiter kann, wie in Figur 44 gezeigt, ein Vorsprung 31 im Endabschnitt eines Stiftes als Schweißlippe dienen. Ein mehrfacher Vorsprung 31 (siehe Figur 41) am Ende des Stiftes kann die Funktion einer Crimplasche oder einer Kühlrippe für einen nachgelagerten Schweißprozess übernehmen.

Eine Ausnehmung 32 kann als Lötstopp dienen und eine Verbreitung des Lots hemmen. Der Vorsprung 31 und/oder die Ausnehmung 32 kann sowohl einzeln, das heißt in Form von einzelnen Nasen oder Mulden oder rundumlaufend in Form eines vorstehenden Stegs oder Kerbe oder Vertiefung ausgeführt sein. Vorzugsweise wird ein Vorsprung 31 oder eine Ausnehmung 32 im Bereich des ersten Schichtelements 1 vorgesehen, diese können sich aber auch in den Bereich des zweiten Schichtelements erstrecken. Der Vorsprung 31 oder die Kerbe 32 können einen runden, einen eckigen oder einen beliebigen (siehe Figur 34) Querschnitt aufweisen. In den Figuren 34, 36 und 43 ist zudem die Lage des Isolators 20 in der Durchführung nach dem Einstecken des Stifts angedeutet. Hieraus ist ersichtlich, dass der Vorsprung 31 den Stift in dem Körper 20 der Durchführung in Position hält. In den Ausführungsvarianten der Figuren 36 und 43 wurde der Vorsprung 31 derart mit einem definierten Querschnitt erzeugt, so dass der Vorsprung 31 geeignet ist, den Körper 20 der Durchführung zu korrekt zu positionieren.

Der Vorsprung 31 und die Ausnehmung 32 kann, wie in den Figuren 44 bis 46 dargestellt, auch in Form einer Verdickung oder Verschmälerung des Stifts ausgeführt werden.

Ferner können in Form von durchgehenden Ausnehmungen 33, wie in den Figuren 32, 33 und 47 gezeigt, Sollbruchstellen vorgesehen werden, welche beim Abreißen des Stifts eine Leckage des elektromedizinischen Implantats verhindern sollen. Die Sollbruchstellen sind dabei in einem Abschnitt des Stifts angeordnet, der so weit außen, das heißt entfernt vom Gehäuseinneren, liegt, dass der hermetische Abschluss des Gehäuses des Implantats weiterhin gewährleistet ist.

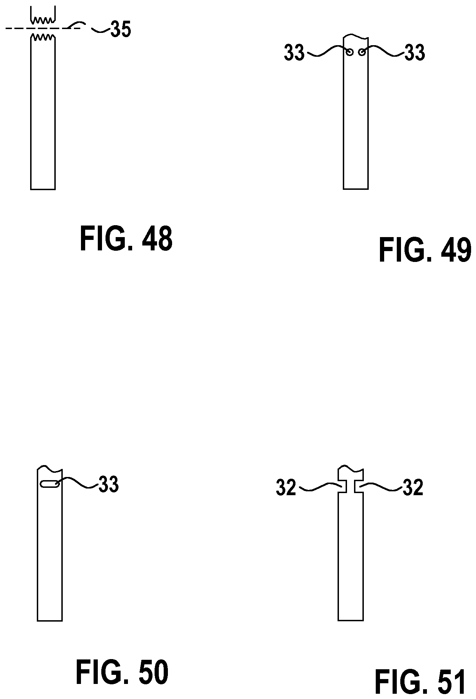

Weitere Sollbruchstellen in Form einer durchgehenden Ausnehmung 33 oder einer Ausnehmung 32 sind in den Figuren 49 bis 51 gezeigt. Diese sollen dazu dienen, den jeweiligen Stift von dem Verbindungssteg zu trennen (angedeutet durch die gestrichelte Linie 35 in Figur 48). Die als durchgehende Ausnehmungen 33 gezeigten Sollbruchstellen können alternativ auch als Einkerbungen ausgeführt sein.

Figur 38 zeigt einen Stift in Form eines Schwertes mit "Griff", "Parierstange" und "Klinge" (Schaft). Die "parierstangenartige" Ausnehmung 32 dient als Auflage und zur Ausrichtung im Körper 20 der Durchführung. Der nach unten gerichtete Fortsatz 31a des Vorsprungs 31 dient zum Ein- beziehungsweise Umgriff in den Körper 20 der Durchführung. Der "Klingenbereich" des Stiftes wird in den Körper 20 der Keramik eingeführt, daher ist dieser vorzugsweise rund ausgeführt. Im Bereich des "Griffes" erfolgt die Anbindung des Headers, vorzugsweise mittels Laserschweißen.

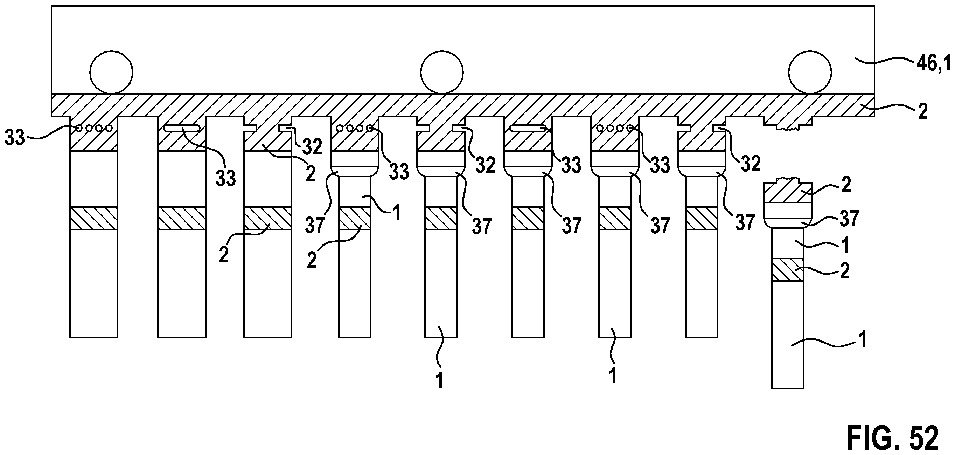

In Figur 52 ist ein Stanzkamm dargestellt, welcher aus dem Halbzeug mit teilweise abgetrennten (z.B. mittels Stanzen freigeschnittenen) Stiften zusammengesetzt ist. Der in der Figur 52 oben liegende Bereich des Halbzeugs bildet einen Verbindungssteg 46. Die in Figur 52 dargestellten Stifte sind beispielhaft in verschiedenen Stadien der Fertigung bzw. mit verschiedenen Ausführungsmöglichkeiten der Sollbruchstelle gezeigt, welche beispielsweise mittels durchgehender Ausnehmungen 33 realisiert ist. Die drei auf der linken Seite dargestellten Stifte zeigen diese nach dem teilweisen Abtrennen (Stanzen) aus dem Halbzeug. Der vierte bis achte Stift (von links gezählt) weisen im Bereich 37 eine Umformung auf und sind nach dem Umformen z.B. mittels erneutem Stanzen gekürzt. Der am weitesten rechts gezeigte Stift wurde entlang der Sollbruchstelle aus dem Stanzkamm herausgelöste und hierdurch von dem Halbzeug vollständig abgetrennt.

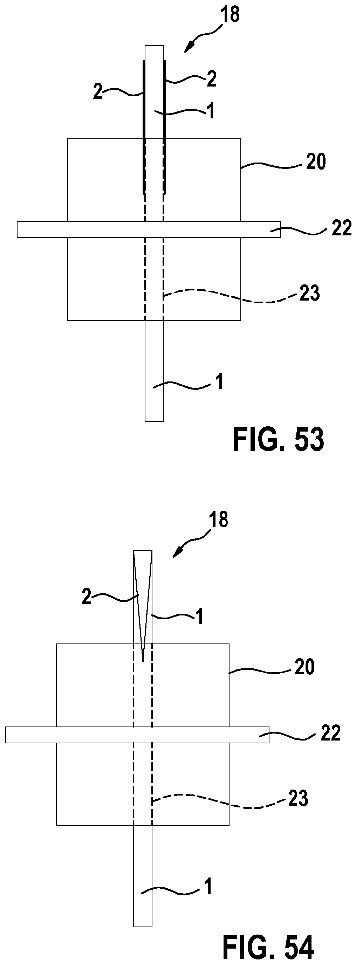

In den Figuren 53 und 54 ist die Anordnung eines erfindungsgemäßen Stifts in einer Durchführung mit einem Körper 20 aus Keramik gezeigt. Der Körper weist einen umlaufenden Flansch 22 zur Anordnung in dem Gehäuse eines Implantats auf. Der Stift sitzt in einer durchgehenden Ausnehmung in Form einer Bohrung 23 in dem Körper 20 wobei die beiden Enden des Stifts in Längsrichtung aus dem Körper 20 hervorstehen. An dem der Platine zugewandten Ende 18 weist der Stift ein zweites Schichtelement 2 in Form eines weichlötbaren Materials auf, das das Löten auf einen Anschluss der Platine erleichtert.

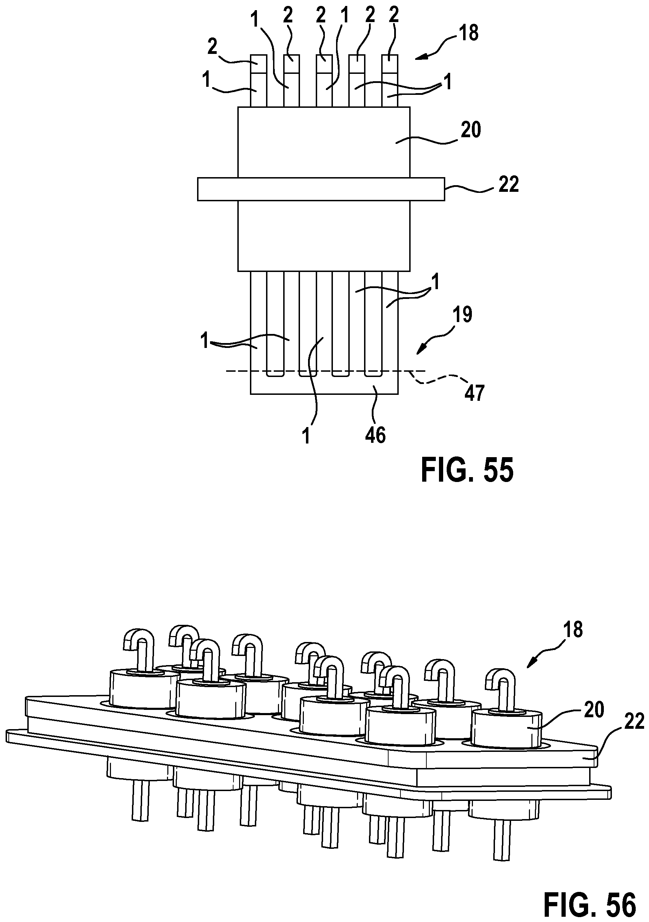

Figur 55 zeigt eine Durchführung mit einem Satz von Stiften, die an dem der Platine abgewandten Ende in dem Endabschnitt 19 mittels eines Verbindungsstegs 46 verbunden sind. Entlang der Trennlinie 47 (gestrichelt) werden nach dem Verlöten der Stifte in dem Körper 20 der Durchführung diese voneinander getrennt. Zur Realisierung des Verbindungsstegs 46 werden die Stifte von dem Halbzeug, wie in Figur 52 gezeigt, nur teilweise abgetrennt, so dass ein Bereich des Halbzeugs als Verbindungssteg 46 stehen bleibt.

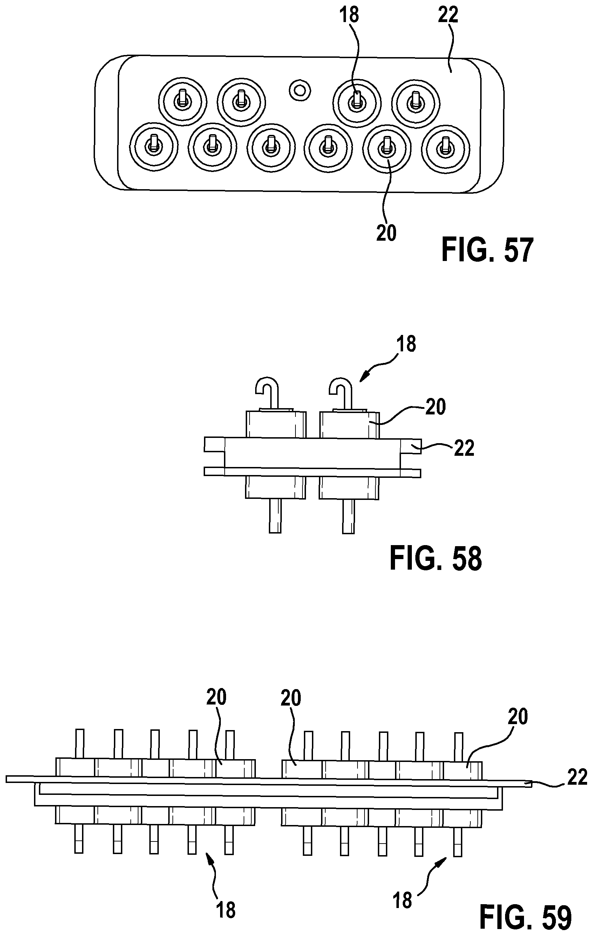

Das weitere Ausführungsbeispiel einer erfindungsgemäßen Durchführung, das in den Figuren 56 bis 59 dargestellt ist, weist in einem Flansch 22, der zur Anordnung der Durchführung in dem elektromedizinischen Implantat dient, und zylinderförmige keramische Körper 20 auf, in die jeweils ein Stift eingelötet ist. Jeder Stift besitzt an seinem, der Platine zugewandtem Ende, einen hakenförmigen Endabschnitt 18, der als J-Lead ausgebildet ist. Der Endabschnitt kann auch dazu genutzt werden, den Stift zur Durchführung zu orientieren und ihn auszurichten. Am Endabschnitt 18 wird der Stift später mit der Platine gefügt. In diesem Bereich weist der Stift, wie in Figur 21 dargestellt ist, ein zweites Schichtelement auf, welches sich gut mit Weichlot benetzen lässt.

Figur 60 veranschaulicht die schrittweise Herstellung eines erfindungsgemäßen Stiftes am Band mittels integrierten Stanzbiegeprozesses. Ausgehend von dem blechförmigen Halbzeug (siehe Schritt A) mit dem blechförmigen ersten Schichtelement 1 aus einem elektrisch leitenden Material und mit diesem aus zwei Bändern gefügten zwei zweiten Schichtelementen 2, die auf dem ersten Schichtelement 1 angeordnet sind und ein Lot oder ein weichlötbares Material enthalten, wird durch Stanzen bzw. Umformen schrittweise der Stift hergestellt. Dazu wird zuerst die Kontur des jeweiligen Stifts von dem Halbzeug teilweise abgetrennt (z.B. mittels Stanzen freigeschnitten, siehe Schritt B), anschließend wird der Schaft 48 des Stifts mittels Umformen rund geformt (siehe Schritt C). Dieser Bereich dient zum Einführen in den Isolator der Keramik und wird daher etwa 0,05 bis 0,4 mm kleiner im Durchmesser sein, als die Bohrung im Isolator um einen fügegerechten Lötspalt zu erzielen. Nach dem Rundprägen muss die Länge des Stifts durch Abtrennen (Ablängen z.B. mittels Stanzen, vergleiche Schritt D) erneut eingestellt werden, da es durch den Umformprozess zu einer Längenänderung in Richtung der Stiftachse kommt. Durch Biegen werden dann der Schaft 48 des Stiftes (in Figur 60 nach unten) und der obere Endabschnitt 49 des Stifts mit dem weichlötbaren zweiten Schichtelement 2 zu einem J-Lead umgeformt (Schritt E). Abschließend kann das überflüssige Material des Halbzeugs entfernt werden (Schritt F). Ein Verbindungssteg 46 in Form eines Gürtels verbindet die fertiggestellten Stifte und dient als Montagehilfe für das gleichzeitige Positionieren von mehreren Bauteilen. Der Gürtel kann an den Sollbruchstellen entfernt werden, welche mittels durchgehender Ausnehmungen 33 oder Materialverjüngungen gebildet wird.

### Bezugszeichenliste

- 1: erstes Schichtelement
- 2: zweites Schichtelement
- 2a, 2b: zweites Schichtelement
- 3: Haftschichtelement
- 4: Antibenetzungsschichtelement
- 5: Trennkante
- 6: Deckschicht
- 8: drittes Schichtelement aus duktilem Metall
- 10: Beschichtung
- 12: Diffusionszone
- 15: Beschichtung
- 17: Endabschnitt des Stifts
- 18: Endabschnitt des Stifts
- 19: Stiftkamm
- 20: Körper der Durchführung
- 22: Flansch
- 23: Bohrung
- 25: Lotkegel
- 31: Vorsprung
- 31a: Fortsatz
- 32: Ausnehmung
- 33: durchgehenden Ausnehmung
- 35: gestrichelte Linie (Abscherkante)
- 37: Bereich mit Umformung
- 46: Verbindungssteg
- 47: Trennlinie
- 48: Schaft des Stifts
- 49: Endabschnitt des Stifts
- A, B: Schritt bei der Herstellung eines erfindungsgemäßen Stifts
- C, D: Schritt bei der Herstellung eines erfindungsgemäßen Stifts
- E, F: Schritt bei der Herstellung eines erfindungsgemäßen Stifts

## Patentansprüche

1. Verfahren zur Herstellung eines Stifts für eine Durchführung für ein elektromedizinisches Implantat mit den folgenden Schritten:
- Erstellen eines folien-, blech- oder bandförmigen Halbzeugs durch Zusammenfügen mindestens eines ersten Schichtelements (1) enthaltend elektrisch leitendes, vorzugsweise biokompatibles, Material in Folien-, Blech- oder Bandform und mindestens eines zweiten Schichtelements (2, 2a, 2b) enthaltend ein Lot und/oder ein weichlötbares Material, vorzugsweise in Draht-, Blech- oder Bandform, oder durch Aufbringen des mindestens einen zweiten Schichtelements auf das mindestens eine erste Schichtelement;
- Aufbringen und gegebenenfalls Fügen mindestens eines Lötinhibitor-Schichtelements (3, 4) enthaltend mindestens ein Element der Gruppe Al, Mg, Ca, Zr, und Y oder eine Legierung aus diesen Elementen in Draht-, Blech- oder Bandform auf das Halbzeug; und
- zumindest teilweises Abtrennen eines Stifts oder eines Satzes mehrerer, mit einem Verbindungssteg (46) verbundener Stifte von dem Halbzeug.

2. Verfahren nach Anspruch 1, **dadurch gekennzeichnet, dass** das erste Schichtelement (1) ein Element der Gruppe Nb, Pt, Pd, Ta, Zr, Ir, Ru und Hf oder einer Legierung daraus, vorzugsweise PtIr10, PtRu10 und/oder medizinischen Edelstahl, enthält.

3. Verfahren nach einem der vorhergehenden Ansprüche, **dadurch gekennzeichnet, dass** das Halbzeug zusätzlich mindestens ein drittes Schichtelement (8) enthaltend einen Isolator oder ein duktiles Metall aufweist, das mit dem mindestens einen ersten Schichtelement und/oder dem mindestens einen zweiten Schichtelement zusammengefügt ist.

4. Verfahren nach einem der vorhergehenden Ansprüche, **dadurch gekennzeichnet, dass** eine Deckschicht (6, 10, 15) zur verbesserten Weichlötbarkeit auf das Halbzeug oder als Schutzschicht, vorzugsweise mittels eines Galvanikbads, vor und/oder nach dem teilweisen Abtrennen des Stifts oder des Satzes mehrerer Stifte von dem Halbzeug aufgebracht wird.

5. Verfahren nach Anspruch 4, **dadurch gekennzeichnet, dass** die Deckschicht (15) zumindest teilweise auf das mindestens eine zweite Schichtelement (2, 2a, 2b) aufgebracht wird.

6. Verfahren nach einem der vorhergehenden Ansprüche, **dadurch gekennzeichnet, dass** der Stift oder mindestens ein Stift des Satzes mehrerer Stifte vor oder nach dem zumindest teilweisen Abtrennen von dem Halbzeug umgeformt wird, vorzugsweise derart, dass das mindestens eine zweite Schichtelement (2, 2a, 2b) den Stift mindestens teilweise umgibt.

7. Verfahren nach Anspruch 6, **dadurch gekennzeichnet, dass** der Stift oder jeder Stift des Satzes mehrerer Stifte nach dem zumindest teilweisen Abtrennen derart umgeformt wird, dass mindestens ein vorzugsweise rundumlaufender Vorsprung (31) und/oder mindestens eine vorzugsweise rundumlaufende Ausnehmung (32) entsteht, wobei der Vorsprung (31) und/oder die Ausnehmung (32) jeweils an der Seitenfläche des jeweiligen Stifts angeordnet ist.

8. Verfahren nach einem der vorhergehenden Ansprüche, **dadurch gekennzeichnet, dass** das zumindest teilweise Abtrennen in eine Richtung im Wesentlichen senkrecht zur Richtung des Zusammenfügens des mindestens einen ersten Schichtelements (1) mit dem mindestens einen zweiten Schichtelement (2, 2a, 2b) und/oder dem mindestens einen dritten Schichtelement erfolgt.

9. Verfahren nach einem der vorhergehenden Ansprüche, **dadurch gekennzeichnet, dass** ein Abschnitt (17) des Stifts nach dem zumindest teilweisen Abtrennen rund umgeformt wird.

10. Verfahren nach einem der vorhergehenden Ansprüche, **dadurch gekennzeichnet, dass** der Stift nach dem zumindest teilweisen Abtrennen an mindestens einem Endabschnitt (18) eine Gullwing, J-Lead oder SOP-ähnliche Form umgeformt wird.

11. Verfahren nach einem der vorhergehenden Ansprüche, **dadurch gekennzeichnet, dass** in einem Endabschnitt (19) eines Stifts des Satzes mehrerer, mit einem Verbindungssteg (46) verbundenen Stifte eine Sollbruchstelle (33) eingebracht wird.

12. Verfahren zur Herstellung einer Durchführung für ein elektromedizinisches Implantat, wobei ein Körper einer Durchführung mit mindestens einer durchgehenden Ausnehmung bereitgestellt wird, weiter die in den in den vorhergehenden Ansprüchen angegebenen Schritte zur Herstellung eines Stifts erledigt werden und der zusätzliche Schritt durchgeführt wird, dass der Stift oder jeder Stift des Satzes mehrerer Stifte anschließend mit der Innenfläche einer durchgehenden Ausnehmung (23) des Isolators (20) mittels Löten verbunden wird.

13. Verfahren nach Anspruch 12, **dadurch gekennzeichnet, dass** die mehreren Stifte anschließend voneinander getrennt werden.

14. Verfahren zur Herstellung eines elektromedizinischen Implantats mit einer Platine und mit den in Anspruch 12 oder 13 angegebenen Schritten zur Herstellung einer Durchführung, wobei anschließend jeder Stift der Durchführung mit einem Anschluss der Platine, vorzugsweise mittels Löten oder Schweißen, verbunden wird.

15. Stift für ein elektromedizinisches Implantat hergestellt mit einem Verfahren nach einem der Ansprüche 1 bis 11.

16. Durchführung für ein elektromedizinisches Implantat hergestellt mit einem Verfahren nach einem der Ansprüche 12 oder 13.

17. Elektromedizinisches Implantat hergestellt mit einem Verfahren nach Anspruch 14.

## Claims

1. A method for producing a pin for a feedthrough for an electromedical implant, the method comprising the following steps:
- creating a film-, sheet- or strip-like semi-finished product by joining at least one first layer element (1) containing an electrically conductive, preferably biocompatible material in film, sheet or strip form and at least one second layer element (2, 2a, 2b) containing a solder and/or a soft-solderable material, preferably in wire, sheet or strip form, or by applying the at least one second layer element to the at least one first layer element;
- applying and, as appropriate, joining at least one solder inhibitor layer element (3, 4) containing at least one element of the group Al, Mg, Ca, Zr and Y or an alloy formed of these elements in wire, sheet or strip form to the semi-finished product; and
- at least partially detaching a pin, or a set of multiple pins connected to a connecting web (46), from the semi-finished product.

2. The method according to claim 1, **characterised in that** the first layer element (1) contains an element of the group Nb, Pt, Pd, Ta, Zr, Ir, Ru and Hf or an alloy formed therefrom, preferably PtIr10, PtRu10 and/or medical stainless steel.

3. The method according to any one of the preceding claims, **characterised in that** the semi-finished product additionally comprises at least one third layer element (8) containing an insulator or a ductile metal, which is joined with the at least one first layer element and/or the at least one second layer element.

4. The method according to any one of the preceding claims, **characterised in that** a top coat (6, 10, 15) is applied to the semi-finished product for improved soft solderability or as a protective layer, preferably by way of an electroplating bath, prior to and/or after the at least partial detachment of the pin, or of the set of multiple pins, from the semi-finished product.

5. The method according to claim 4, **characterised in that** the top coat (15) is applied to at least a portion of the at least one second layer element (2, 2a, 2b).

6. The method according to any one of the preceding claims, **characterised in that** the pin, or at least one pin of the set of multiple pins, is formed prior to or after being at least partially detached from the semi-finished product, preferably in such a way that the at least one second layer (2, 2a, 2b) at least partially surrounds the pin.

7. The method according to claim 6, **characterised in that** the pin, or each pin of the set of multiple pins, is reshaped after the at least partial detachment in such a way that at least one preferably circumferential protrusion (31) and/or at least one preferably circumferential recess (32) are/is created, wherein the protrusion (31) and/or the recess (32) are/is located in each case on the side surface of the pin in question.

8. The method according to any one of the preceding claims, **characterised in that** the at least partial detachment takes place in a direction that is substantially perpendicular to the direction of joining of the at least one first layer element (1) to the at least one second layer element (2, 2a, 2b) and/or the at least one third layer element.

9. The method according to any one of the preceding claims, **characterised in that** a portion (17) of the pin is converted into a round shape after the at least partial detachment.

10. The method according to any one of the preceding claims, **characterised in that** the pin is formed on at least one end portion (18) into a gull wing, J-lead or SOP-like shape after the at least partial detachment.

11. The method according to any one of the preceding claims, **characterised in that** a predetermined breaking point (33) is introduced into an end portion (19) of a pin of the set of multiple pins connected to a connecting web (46).

12. A method for producing a feedthrough for an electromedical implant, wherein a body of a feedthrough having at least one continuous recess is provided, and wherein the steps for producing a pin as specified in the preceding claims are performed and an additional step is carried out, according to which the pin, or each pin of the set of multiple pins, is subsequently connected to the inner surface of a continuous recess (23) of the insulator (20) by way of soldering.

13. The method according to claim 12, **characterised in that** the multiple pins are subsequently detached from each other.

14. A method for producing an electromedical implant comprising a printed circuit board and with the steps specified in claim 12 or 13 for producing a feedthrough, wherein subsequently each pin of the feedthrough is connected to a terminal of the printed circuit board, preferably by way of soldering or welding.

15. A pin for an electromedical implant, produced using a method according to any one of clams 1 to 11.

16. A feedthrough for an electromedical implant, produced by a method according to either one of claims 12 or 13.

17. An electromedical implant, produced using a method according to claim 14.

## Revendications

1. Procédé de fabrication d'une broche pour une traversée destinée à un implant électromédical avec les étapes suivantes:
- la fabrication d'un demi-produit en forme de film, de plaque ou de bande par l'assemblage d'au moins un premier élément stratifié (1) contenant de la matière électriquement conductrice, de préférence biocompatible, en forme de film, de plaque ou de bande et d'au moins un deuxième élément stratifié (2, 2a, 2b) contenant une matière de brasage et/ou pouvant être brasée de manière tendre, de préférence, en forme de fil, de plaque ou de bande, ou par l'applicationde l'au moins un deuxième élément stratifié sur l'au moins un premier élément stratifié;
- l'application, et éventuellement l'assemblage, d'au moins un élément stratifié d'inhibiteur de brasage (3, 4) contenant au moins un élément du groupe Al, Mg, Ca, Zr, et Y ou un alliage à base de ces éléments en forme de fil, de plaque ou de bande sur le demi-produit; et
- la séparation au moins partielle d'une broche ou d'un jeu de plusieurs broches reliées avec une barrette de liaison (46) du demi-produit.

2. Procédé selon la revendication 1, **caractérisé en ce que** le premier élément stratifié (1) contient un élément du groupe Nb, Pt, Pd, Ta, Zr, Ir, Ru et Hf ou un alliage de ceux-ci, de préférence PtIr10, PtRu10 et/ou un acier inoxydable médical.

3. Procédé selon l'une des revendications précédentes, **caractérisé en ce que** le demi-produit présente au moins un troisième élément stratifié (8) contenant un isolant ou un métal ductile, qui est assemblé avec l'au moins un premier élément stratifié et/ou avec l'au moins un deuxième élément stratifié.

4. Procédé selon l'une des revendications précédentes, **caractérisé en ce qu'**une couche de revêtement (6, 10, 15) est appliquée sur le demi-produit pour un brasage tendre amélioré ou en tant que couche de protection, de préférence au moyen d'un bain de galvanisation, avant et/ou après la séparation partielle de la broche ou du jeu de plusieurs broches du demi-produit.

5. Procédé selon la revendication 4, **caractérisé en ce que** la couche de revêtement (15) est appliquée au moins partiellement sur l'au moins un deuxième élément stratifié (2, 2a, 2b).

6. Procédé selon l'une des revendications précédentes, **caractérisé en ce que** la broche ou l'au moins une broche du jeu de plusieurs broches est modifiée avant ou après l'au moins une séparation partielle du demi-produit, de préférence de telle manière que l'au moins un deuxième élément stratifié (2, 2a, 2b) entoure au moins partiellement la broche.

7. Procédé selon la revendication 6, **caractérisé en ce que** la broche ou chaque broche du jeu de plusieurs broches est modifiée après l'au moins une séparation partielle de telle manière qu'au moins une saillie (31) de préférence parcourant la circonférence, et/ou au moins une cavité (32) de préférence parcourant la circonférence, est créée, où la saillie (31), et/ou la cavité (32), est disposée respectivement sur une surface latérale de la broche respective.

8. Procédé selon l'une des revendications précédentes, **caractérisé en ce que** l'au moins une séparation partielle a lieu dans une direction essenteillement perpendiculaire à la direction de l'assemblage de l'au moins un premier élément stratifié (1) avec l'au moins un deuxième élément stratifié (2, 2a, 2b) et/ou l'au moins un troisième élément stratifié.

9. Procédé selon l'une des revendications précédentes, **caractérisé en ce qu'**une partie (17) de la broche est modifiée en une forme arrondie après l'au moins une séparation partielle.

10. Procédé selon l'une des revendications précédentes, **caractérisé en ce que** la broche est modifiée après l'au moins une séparation partielle au niveau d'une extrémité (18) en forme d'aile de mouette, de connecteur J ou une forme similaire à une procédure opérationnelle permanente SOP.

11. Procédé selon l'une des revendications précédentes, **caractérisé en ce qu'**un point de rupture souhaité (33) est appliqué au niveau d'une d'extrémité (19) d'une broche du jeu de plusieurs broches reliées avec une barrette de liaison (46).

12. Procédé de fabrication d'une traversée pour un implant électromédical, où un corps d'une traversée est préparé avec au moins un évidement traversant, puis, les étapes indiquées dans les revendications précédentes pour la fabrication d'une broche sont exécutées et l'étape supplémentaire est exécutée, dans laquelle la broche ou chaque broche du jeu de plusieurs broches est ensuite reliée exclusivement avec la surface intérieure d'un évidement traversant (23) de l'isolant (20) au moyen d'un brasage.

13. Procédé selon la revendication 12, **caractérisé en ce que** les multiples broches sont ensuite séparées les unes des autres.

14. Procédé de fabrication d'un implant électromédical avec un circuit imprimé et avec les étapes indiquées dans la revendication 12 ou 13 pour la fabrication d'une traversée, où, ensuite, chaque broche de la traversée est reliée avec une borne du circuit imprimé, de préférence, au moyen d'un brasage ou d'une soudure.

15. Broche pour un implant électromédical fabriquée avec le procédé selon l'une des revendications 1 à 11.

16. Traversée pour un implant électromédical fabriquée avec un procédé selon l'une des revendications 12 ou 13.

17. Implant électromédical fabriqué avec un procédé selon la revendication 14.
